# EUROPÄISCHE PATENTANMELDUNG

(11) **EP 1 982 985 A2**
(43) Veröffentlichungstag der Anmeldung: **22.10.2008**
(21) Anmeldenummer: 08161021.4
(22) Anmeldetag: 24.11.1998
(51) Int. Cl.: C07D 473/32

(54) **Verfahren zur Herstellung eines Purinderivats**

(30) Priorität: 27.11.1997 CH 273997; 21.01.1998 CH 1331998; 27.03.1998 CH 7231998; 07.10.1998 EP 98118895; 03.12.1997 CH 278197
(62) Teilanmeldung aus: 06002571.5
(71) Anmelder: Lonza AG, 3930 Visp (CH)
(72) Erfinder: Berchtold, Katja, 3937, Baltschieder (CH); Roduit, Jean-Paul, 1950, Sion (CH); Bernegger-Egli, Christine, 3985, Münster (CH); Urban, Eva Maria, 3912, Termen (CH); Petersen, Michael, 79540, Lörrach (DE)
(74) Vertreter: Winter, Brandl, Fürniss, Hübner Röss, Kaiser, Polte Partnerschaft Patent- und Rechtsanwaltskanzlei

(57) **Zusammenfassung**

Beschrieben wird ein Verfahren zur Herstellung von (1S,4R)-4-(2-amino-6-chlor-9-H-purin-9-yl)-2-cyclopenten-1-methanol der Formel 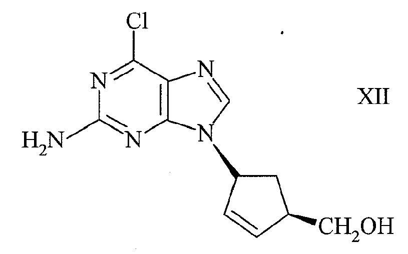 Dabei wird ein Aminoalkohol der Formel 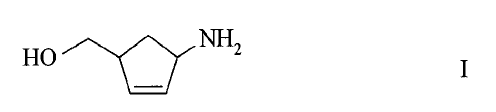 oder ein Cyclopentenderivat der allgemeinen Formel 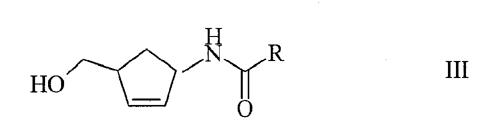 worin R C₁-₄-Alkyl, C₁-₄-Alkoxy, Aryl oder Aryloxy bedeutet, mit N-(2-amino-4,6-dichlorpyrimidin)5-yl)formamid der Formel 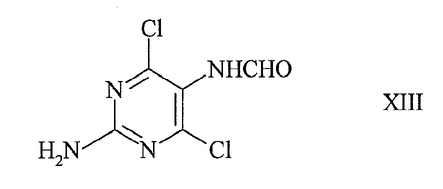 in das (1 S,4R)-4-[(2-Amino-6-chlor-5-formamido-4-pyrimidinyl)-amino]-2-cyclopenten-1-methanol der Formel 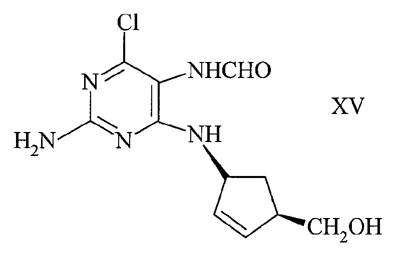 überführt, das dann zur Endverbindung cyclisiert wird.

## Beschreibung

Die vorliegende Erfindung betrifft ein Verfahren zur Herstellung von (1S,4R)-4-(2-Amino-6-chlor-9-H-purin-9-yl)-2-cyclopenten-1-methanol der Formel 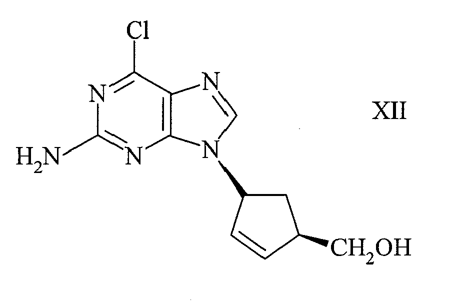 oder dessen Salzen.

(1R,4S)-1-Amino-4-(hydroxymethyl)-2-cyclopenten ist ein wichtiges Zwischenprodukt zur Herstellung von carbocyclischen Nukleosiden wie z. B. Carbovir^{®} (Campbell et al., J. Org. Chem. 1995, 60, 4602 - 4616). Ein Verfahren zur Herstellung von (1R,4S)-1-Amino-4-(hydroxymethyl)-2-cyclopenten ist beispielsweise von Campbell et al. (ibid) und von Park K. H. & Rapoport H. (J. Org. Chem. 1994, 59, 394 - 399) beschrieben. Bei diesem Verfahren dient als Edukt entweder D-glucon-δ-lacton oder D-Serin, wobei ca. 15 Synthesestufen bis zur Bildung von (1R,4S)-N-tert-Butoxycarbonyl-4-hydroxymethyl-2-cyclopenten, welches dann zum (1R,4S)-1-Amino-4-(hydroxymethyl)-2-cyclopenten deprotektioniert wird, notwendig sind. Diese beiden Verfahren sind kostspielig, umständlich und grosstechnisch nicht gangbar. Die WO 93/17020 beschreibt ein Verfahren zur Herstellung von (1R,4S)-1-Amino-4-(hydroxymethyl)-2-cyclopenten, wobei (1R,4S)-4-Amino-2-cyclopenten-1-carbonsäure mit Lithiumaluminiumhydrid zum gewünschte Produkt reduziert wird.
Nachteilig bei diesem Verfahren ist zum einen, dass auch die Doppelbindung des Cyclopentenringes reduziert wird, die schlechte Handhabbarkeit von Lithiumaluminiumhydrid und zum anderen, dass es zu kostspielig ist.
Taylor S. J. et al. (Tetrahetron: Asymmetry Vol. 4, No. 6, 1993, 1117 - 1128) beschreiben ein Verfahren zur Herstellung von (1R,4S)-1-Amino-4-(hydroxymethyl)-2-cyclopenten ausgehend von (±)-2-Azabicyclo[2.2.1]hept-5-en-3-on als Edukt. Dabei wird das Edukt mittels Mikroorganismen der Spezies Pseudomonas solanacearum oder Pseudomonas fluorescens in (1R,4S)-2-Azabicyclo[2.2.1]hept-5-en-3-on überführt, welches dann mit Di-tert-butyldicarbonat in das (1R,4S)-N-tert-butoxycarbonyl-2-azabicyclo[2.2.1]hept-5-en-3-on umgesetzt wird. Letzteres mit Natriumborhydrid und Trifluoressigsäure zum gewünschten Produkt reduziert. Auch dieses Verfahren ist viel zu kostspielig.

Desweiteren beschreiben Martinez et al. (J. Org. Chem. 1996, 61, 7963 - 7966) eine 10stufige Synthese von (1R,4S)-1-Amino-4-(hydroxymethyl)-2-cyclopenten ausgehend von Dialkylmalonsäurediethylester. Auch dieses Verfahren hat den Nachteil, dass es umständlich und grosstechnisch nicht gangbar ist.
Bekannt ist auch, dass man N-substituierte-(±)-2-Azabicyclo[2.2.1]hept-5-en-3-one, welche einen elektronenziehenden Substituenten tragen, mit einem Metallhydrid zu den entsprechenden N-substituierten Aminoalkoholen reduzieren kann (Katagiri et al., Tetrahedron Letters, 1989, 30, 1645 - 1648; Taylor et al., ibid).
Im Gegensatz dazu ist bekannt, dass unsubstituiertes (±)-2-Azabicyclo[2.2.1]hept-5-en-3-on der Formel 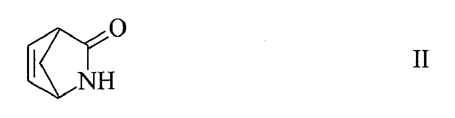 mit Lithiumaluminiumhydrid zu (±)-2-Azabicyclo[2.2.1]octen reduziert wird (Malpass & Tweedle, J. Chem. Soc., Perkin Trans 1, 1977, 874 - 884) und dass die direkte Reduktion von (±)-2-Azabicyclo[2.2.2]hept-5-en-3-on zum entsprechenden Aminoalkohol bislang als nicht möglich galt (Katagiri et al., ibid; Taylor et al., ibid).

Bekannt ist auch, racemisches 1-Amino-4-(hydroxymethyl)-2-cyclopenten mittels (-)- Dibenzoylweinsäure zu spalten (US-A 5 034 394). Diese Umsetzung hat einerseits den Nachteil, dass die (-)-Dibenzoylweinsäure teuer ist, anderseits, dass die Trennung in Gegenwart eines genau definierten Gemisches von Acetonitril und Ethanol erfolgen muss. Dieses Lösungsmittelgemisch kann nicht getrennt werden und muss der Verbrennung zugeführt werden.

Aufgabe der vorliegenden Erfindung war es, ein einfaches, ökonomisches und kostengünstiges Verfahren zur Herstellung von (1R,4S)-1-Amino-4-(hydroxymethyl)-2-cyclopenten zur Verfügung zu stellen.

Überraschenderweise wurde nun gefunden, dass wenn man (±)-2-Azabicyclo[2.2.1]hept-5-en-3-on der Formel 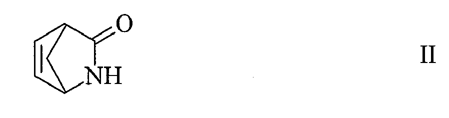 in Form des Racemats oder eines seiner optisch aktiven Isomere, mit einem Metallhydrid reduziert, der Aminoalkohol der Formel 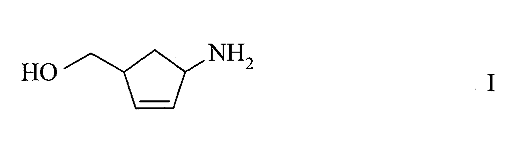 in Form des Racemats oder eines seiner optisch aktiven Isomere, auf einfache Weise erhalten wird.
Vorzugsweise wird der racemische cis-Aminoalkohol der Formel I erhalten.

Wie fachmännisch üblich kann der Aminoalkohol der Formel I mit einer Säure in die entsprechenden Salze überführt werden, wie beispielsweise in Hydrohalogenidsalze. Als Hydrohalogenidsalze sind Hydrobromide und Hydrochloride geeignet.

Das Edukt, das (±)-2-Azabicyclo[2.2.1]hept-5-en-3-on, kann gemäss der EP-A 0 508 352 hergestellt werden.

Als Metallhydride können Alkalimetall- oder Erdalkalimetallhydride sowie binäre oder komplexe Metallhydride der Bor- oder Aluminiumgruppe eingesetzt werden, beispielsweise Alkalimetall- und Erdalkalimetallborhydride und Alkalimetall- und Erdalkalimetallaluminiumhydride.

Als Alkalimetall- oder Erdalkalimetallhydride sind LiH, NaH, KH, BeH₂, MgH₂ oder CaH₂ geeignet. Als binäre Alkalimetall- oder Erdalkalimetallborhydride können NaBH₄, LiBH₄, KBH₄, NaAlH₄, LiAlH₄, KAlH₄, Mg(BH₄)₂, Ca(BH₄)₂, Mg(AlH₄)₂, Ca(AlH₄)₂ verwendet werden. Komplexe Metallhydride der Bor- oder Aluminiumgruppe können die allgemeine Formel M¹ M² HₙLₘ haben, worin n eine ganze Zahl von 1 bis 4 ist und m eine ganze Zahl von 4 bis 4 minus der entsprechenden Zahl n ist, M¹ ein Alkalimetallatom bedeutet, M² Bor oder Aluminium bedeutet und L C₁₋₄-Alkyl, C₁₋₄-Alkenyl, C₁₋₄-Alkoxy, CN oder ein Amin ist oder die komplexen Metallhydride können die allgemeine Formel M²H_{O}Lₚ haben, worin M² die genannte Bedeutung hat und O eine ganze Zahl von 0 bis 3 ist und p eine ganze Zahl von 3 bis 3 minus der entsprechenden Zahl p ist. Als M¹ M² HₙLₘ können LiBH (C₂H₅)₃, LiBHₓ (OCH₃)₄₋ₓ, worin x eine ganze Zahl von 1 bis 3 bedeutet, LiAlH(OC(CH₃)₃)₃, NaAlH₂(OC₂H₄OCH₃)₂, NaAlH₂(C₂H₅)₂ oder NaBH₃CN eingesetzt werden. Vorzugsweise wird die Reduktion mit einem Metallborhydrid durchgeführt.
Wie fachmännisch bekannt, können die erwähnten Metallhydride wie z. B. LiBH₄ auch "in situ" erzeugt werden. Gängige Darstellungsmethoden für LiBH₄ sind beispielsweise die Umsetzung eines Alkalimetallborhydrids mit einem Lithiumhalogenid (H. C. Brown et al., Inorg. Chem. 20, 1981, 4456 - 4457), die Umsetzung von LiH mit B₂O₃ in Gegenwart von Wasserstoff und einem Hydrierkatalysator (EP-A 0 512 895), die Umsetzung von LiH mit (H₅C₂)OBF₃ (DE-OS 94 77 02) und die von LiH mit B(OCH₃)₃ (US-A 2,534,533).

Zweckmässig werden die Metallhydride in einem molaren Verhältnis von 1 bis 5 pro mol (±)-2-Azabicyclo[2.2.1]hept-5-en-3-on eingesetzt.

Vorzugsweise werden die Metallhydride, insbesondere NaBH₄, mit Lithiumsalzzusätzen verwendet. Als Lithiumsalze können LiCl, LiBr, LiF, LiJ, L₂SO₄, LiHSO₄, Li₂CO₃, Li(OCH₃) und LiCO₃ verwendet werden.

Zweckmässig wird die Reduktion unter Inertgasatmosphäre, beispielsweise unter Argon- oder Stickstoffatmosphäre, durchgeführt.

Die Reduktion kann bei einer Temperatur von -20 bis 200 °C, vorzugsweise bei einer Temperatur von 60 bis 150 °C, durchgeführt werden.

Als Lösungsmittel sind aprotische oder protische organische Lösungsmittel geeignet. Als aprotische organische Lösungsmittel können Ether oder Glykolether wie beispielsweise Diethylether, Dibutylether, Ethylmethylether, Diisopropylether, tert-Butylmethylether, Anisol, Dioxan, Tetrahydrofuran, Mono-Glyme, Diglyme und Formaldehyddimethylacetal eingesetzt werden. Als protische organische Lösungsmittel sind C₁₋₆-Alkohole wie Methanol, Ethanol, Propanol, Isopropanol, Butanol, tert-Butanol, Pentanol, tert-Amylalkohol oder Hexanol sowie Mischungen von diesen mit Wasser geeignet. Als protische organische Lösungsmittel ebenso geeignet sind Mischungen von einem der genannten Ether, Glykolether mit Wasser oder mit einem der genannten Alkohole wie eine Mischung von einem C₁₋₆-Alkohol mit einem Ether oder Glykolether, insbesondere eine Mischung von Methanol, Ethanol oder Wasser mit Diethylether, Tetrahydrofuran, Dioxan, Glyme oder Diglyme. Vorzugsweise wird als Lösungsmittel ein protisches organisches Lösungsmittel, wie eine Mischung von einem C₁₋₆-Alkohol oder Wasser mit einem Ether oder Glykolether, eingesetzt.

In einer bevorzugten Ausführungsform wird die Reduktion in Gegenwart eines Zusatzes, beispielsweise in Gegenwart von Wasser oder eines ein- oder mehrwertigen C₁₋₆-Alkohols durchgeführt. Als einwertige C₁₋₆-Alkohol können Methanol, Ethanol, Methoxyethanol, n-Propanol, Isopropanol, Isobutanol, tert-Butanol, n-Butanol verwendet werden. Als mehrwertige Alkohole können bspw. Diole wie Butandiol und Triole wie Glycerin verwendet werden. Insbesondere wird als C₁₋₆-Alkohol Methanol oder Ethanol verwendet. Zweckmässig wird dabei der C₁₋₆-Alkohol in einem molaren Verhältnis von 2 bis 15 pro mol (±)-2-Aza-bicyclo[2.2.1]hept-5-en-3-on eingesetzt.

Wird die Umsetzung in Gegenwart der genannten Alkohole durchgeführt, kann in situ (intermediär) der entsprechende Aminosäureester gebildet werden. D. h. wird als Edukt (+)-2-Azabicyclo[2.2.1]hept-5-en-3-on verwendet, kann erfindungsgemäss der entsprechende (+)-Aminosäureester gebildet werden.

Wird als Edukt (-)-2-Azabicyclo[2.2.1]hept-5-en-3-on verwendet, kann erfindungsgemäss dementsprechend der (-)-Aminosäureester intermediär gebildet werden.
Überraschenderweise wurde auch gefunden, dass wenn man ein Cyclopentenderivat der allgemeinen Formel 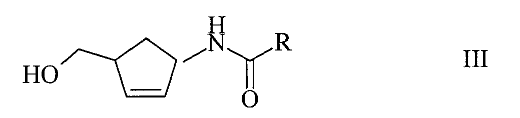 in Form des Racemats oder eines seiner optisch aktiven Isomere, worin R C₁₋₄-Alkyl, C₁₋₄-Alkoxy, Aryl oder Aryloxy bedeutet, mit einem Alkalimetallhydroxid hydrolysiert der Aminoalkohol der Formel 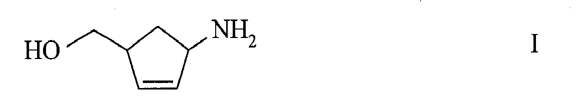 in Form des Racemats oder eines seiner optisch aktiven Isomere auf einfache Weise erhalten wird.

C₁₋₄-Alkyl kann substituiert oder unsubstituiert sein. Unter substituiertem C₁₋₄-Alkyl wird im folgenden mit einem oder mehreren Halogenatomen substituiertes C₁₋₄-Alkyl verstanden. Als Halogenatom kann F, Cl, Br oder J angewendet werden. Beispiele für C₁₋₄-Alkyl sind Methyl, Ethyl, Propyl, Butyl, Isobutyl, tert. Butyl, Isopropyl, Chlormethyl, Brommethyl, Dichlormethyl, Dibrommethyl. Vorzugsweise wird als C₁₋₄-Alkyl Methyl, Ethyl, Propyl, Butyl, Isobutyl oder Chlormethyl verwendet.

Als C₁₋₄-Alkoxy kann beispielsweise Methoxy, Ethoxy, Propoxy oder Butoxy verwendet werden. Als Aryl kann beispielsweise Phenyl oder Benzyl, substituiert oder unsubstituiert, verwendet werden. Als substituiertes Phenyl oder Benzyl wird im folgenden mit einem oder mehreren Halogenatomen substituiertes Phenyl oder Benzyl verstanden, wie bspw. Chlorbenzyl, Dichlorbenzyl, Bromphenyl oder Dibromphenyl.

Als Aryloxy kann beispielsweise Benzyloxy oder Phenoxy, substituiert mit den zuvor beschriebenen Substituenten oder unsubstituiert, verwendet werden.

Als Alkalimetallhydroxid kann Natrium- oder Kaliumhydroxid eingesetzt werden. Für diese Verfahrensvariante wird bevorzugt das Cyclopentenderivat der allgemeinen Formel III durch Reduktion von dem entsprechenden Acyl-2-Azabicyclo[2.2.1]-hept-5-en-3-on der allgemeinen Formel 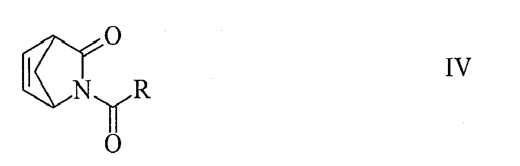 in Form des Racemats oder eines seiner optisch aktiven Isomeren, worin R die genannte Bedeutung hat, mit einem der bereits erwähnten Metallhydride in einem wasserfreien Lösungsmittel hergestellt.

Als wasserfreies Lösungsmittel können protische oder aprotische organische Lösungsmittel eingesetzt werden, insbesondere ein wasserfreies protisches organisches Lösungsmittel wie ein tertiärer Alkohol. Als tertiärer Alkohol kann beispielsweise tert-Butylalkohol oder tert-Amylalkohol verwendet werden.

Wie bereits zuvor beschrieben, wird auch diese Reduktion bevorzugt in Gegenwart eines Zusatzes, beispielsweise in Gegenwart eines C₁₋₆-Alkohols wie Methanol, insbesondere in Gegenwart von 2 mol Methanol pro mol Acyl-2-Azabicyclo[2.2.1]-hept-5-en-3-on (Formel IV), durchgeführt.

Zweckmässig wird die Umsetzung bei einer Temperatur von 0 bis 50 °C, vorzugsweise von 15 bis 30 °C durchgeführt.

Der racemische, bevorzugt der cis-racemische, Aminoalkohol der Formel I wird dann erfindungsgemäss entweder chemisch mit einer optisch aktiven Weinsäure oder auf biotechnologische Weise mittels einer Hydrolase in Gegenwart eines Acylierungsmittels in das (1R,4S)- oder (1S,4R)-1-Amino-4-(hydroxymethyl)-2-cyclopenten der Formel 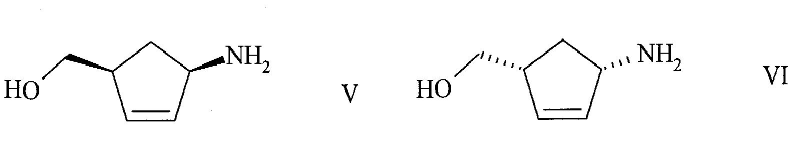 bzw. deren Salze und/oder in das (1S,4R)- oder (1R,4S)-1-Amino-4-(hydroxymethyl)-2-cyclopentenderivat der allgemeinen Formeln 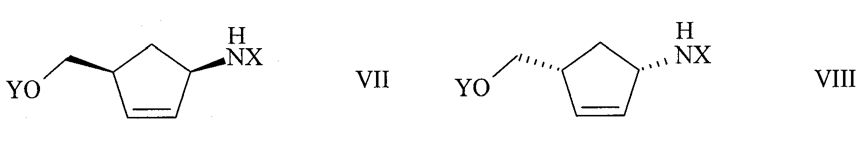 bzw. deren Salze, worin X und Y gleich oder verschieden sind und eine Acylgruppe oder H bedeuten, ausgenommen X = Y = H, umgesetzt.

Als Hydrolasen können Lipasen, Proteasen, Amidasen oder Esterasen eingesetzt werden, zweckmässig werden Lipasen eingesetzt.

Unter Salzen werden im folgenden Hydrohalogenidsalze wie Hydrochloride, Hydrobromide oder Tartrate verstanden.

Wie fachmännisch bekannt, können hydrolasekatalysierte Acylierungen, bei denen optisch aktive Verbindungen gebildet werden, in Gegenwart eines geeigneten Acylierungsmittels durchgeführt werden (Balkenhohl et al., 1997, J. Prakt. Chem. 339, 381 - 384; K. Faber, "Biotransformation in Organic Chemistry", 2nd ed., Berlin 1995, 270 - 305). Als Acylierungsmittel sind generell Carbonsäurederivate wie Carbonsäureamide, Carbonsäureanhydride oder Carbonsäureester geeignet. Als Carbonsäureester können beispielsweise Alkoxycarbonsäureester wie Methoxyessigsäureethylester und Methoxyessigsäureisopropylester, C₁₋₆-Carbonsäureester wie Essigsäurebutylester, Buttersäureethylester und Hexansäureethylester, Glycerinester wie Tributyrin (Glycerintributyrat), Glykolester wie Glykoldibutyrat und Diglykolsäurediethylester, Dicarbonsäureester wie Fumar- und Malonsäurediethylester, Cyancarbonsäureester wie Cyanessigsäureethylester oder cyclische Ester wie beispielsweise 6-Caprolacton verwendet werden. Demzufolge entspricht die Acylgruppe in den Formeln VII und VIII der Säurekomponente des eingesetzten Carbonsäurederivats.

### Als Lipase können handelsübliche Lipasen verwendet werden wie beispielsweise:

Novo-Lipase SP523 aus Aspergillus oryzae (Novozym 398), Novo-Lipase SP524 aus Aspergillus oryzae (Lipase = Palatase 20000L von Novo), Novo-Lipase SP525 aus Candida antarctica (Lipase B Novozym 435, immobilisiert), Novo-Lipase SP526 aus Candida antarctica (Lipase A = Novozym 735, immobilisiert), Lipase-kits von Fluka (1 & 2), Amano P Lipase, Lipase aus Pseudomonas sp., Lipase aus Candida cylindracea, Lipase aus Candida lipolytica, Lipase aus Mucor miehei, Lipase aus Aspergillus niger, Lipase aus Bacillus thermocatenulatus, Lipase aus Candida antarctica, Lipase AH (Amano; immobilisiert), Lipase P (Nagase), Lipase AY aus Candida rugosa, Lipase G (Amano 50), Lipase F (Amano F-AP15), Lipase PS (Amano), Lipase AH (Amano), Lipase D (Amano), Lipase AK aus Pseudomonas fluorescens, Lipase PS aus Pseudomonas cepacia, Newlase I aus Rhizopus niveus, Lipase PS-CI (immobilisierte Lipase aus Pseudomonas cepacia).
Diese Lipasen können, wie fachmännisch bekannt, als zellfreie Enzymextrakte oder auch in der entsprechenden Mikroorganismenzelle angewendet werden.

Als Proteasen können ebenfalls handelsübliche verwendet werden wie beispielsweise Serinproteasen wie Subtilisine. Als Subtilisin kann Savinase aus Bacillus sp., Alcalase, Subtilisin aus Bacillus licheniformis sowie Proteasen aus Aspergillus, Rhizopus, Streptomyces oder Bacillus sp. verwendet werden.
Zweckmässig wird die biotechnologische Racematspaltung bei einer Temperatur von 10 bis 80 °C und bei einem pH von 4 bis 9 durchgeführt.

Die biotechnologische Racematspaltung wird zweckmässig in einem protischen oder aprotischen organischen Lösungsmittel durchgeführt. Als aprotische organische Lösungsmittel sind Ether wie tert-Butylmethylether, Diisopropylether, Dibutylether, Dioxan und Tetrahydrofuran, aliphatische Kohlenwasserstoffe wie Hexan, organische Basen wie Pyridine und Carbonsäureester wie Ethylacetat und als protische organische Lösungsmittel die bereits beschriebenen C₁₋₆-Alkohole wie z. B. Pentanol geeignet.

Die bei der biotechnologischen Racematspaltung erfindungsgemäss gebildeten (1S,4R)- oder (1R,4S)-1-Amino-4-(hydroxymethyl)-2-cyclopentenderivate der allgemeinen Formeln VII und VIII werden, abhängig von der gewünschten Zielverbindung (Aminoalkohol der Formel V oder VI), auf chemische Weise zum Aminoalkohol der Formel V oder VI hydrolysiert. Die chemische Hydrolyse erfolgt zweckmässig in einer wässrigen basischen Lösung oder mittels eines basischen Ionenaustauschers. Als wässrige basische Lösung wird vorzugsweise wie für die zuvor beschriebene Hydrolyse der Cyclopentenderivate der allgemeinen Formel III ein Alkalimetallhydroxid eingesetzt. Als basische Ionenaustauscher können beispielsweise Dowex 1x8(OH⁻) und Duolite A147 eingesetzt werden.

Die chemische Racematspaltung wird mit einer optisch aktiven Weinsäure wie mit D-(-)-Weinsäure oder L-(+)-Weinsäure durchgeführt.

Die Racematspaltung mit D-(-)-Weinsäure wird zweckmässig so durchgeführt, dass das racemische 1-Amino-4-(hydroxymethyl)-2-cyclopenten zunächst mit der D-(-)-Weinsäure in Gegenwart eines C₁₋₆-Alkohols zur Umsetzung gebracht wird. Geeignete C₁₋₆-Alkohole sind die gleichen wie die zuvor beschriebenen. Bevorzugt wird Methanol eingesetzt.
Die Umsetzung, die zur Salzbildung führt, wird üblicherweise bei einer Temperatur zwischen 20°C und Rückflusstemperatur des Lösungsmittels, vorzugsweise bei Rückflusstemperatur durchgeführt.
Wenn gewünscht, kann das hierbei entstehende 1-Amino-4-(hydroxymethyl)-2-cyclopenten-D-tartrat durch Umkristallisation aus einem C₁₋₆-Alkohol wie Methanol weiter gereinigt werden.

Die Racematspaltung mit L-(+)-Weinsäure wird zweckmässig analog zu der mit D-(-)-Weinsäure durchgeführt. D.h., dass die Racematspaltung mit L-(+)-Weinsäure ebenfalls in Gegenwart eines C₁₋₆-Alkohols und bei einer Temperatur zwischen 20 °C und Rückflusstemperatur des Lösungsmittels, vorzugsweise bei Rückflusstemperatur durchgeführt werden kann. Nach Abkühlen kristallisiert das (1S,4R)-1-Amino-4-(hydroxymethyl)-2-cyclopenten-hydrogen-L-tartrat aus.
Das (1R,4S)-1-Amino-4-(hydroxymethyl)-2-cyclopenten hydrogen-L-tartrat liegt insbesondere gelöst in der Mutterlauge vor.
Die Isolation, die weitere Reinigung (Freisetzung) sowie die Umsetzung zum entsprechenden Salz des (1R,4S)- oder (1S,4R)-1-Amino-4-(hydroxymethyl)-2-cyclopentens erfolgt mit einer Base und anschliessender Säurebehandlung. Geeignete Basen sind Alkalimetallalkoholate, Alkalimetall- oder Erdalkalimetallcarbonate und Alkalimetall- oder Erdalkalimetallhydroxide. Als Alkalimetallalkoholate können Natrium- oder Kaliumalkoholate eingesetzt werden. Als Alkalimetallcarbonat kann Kalium- oder Natriumcarbonat, Kalium- oder Natriumhydrogencarbonat und als Erdalkalimetallcarbonat kann Magnesium- oder Calciumcarbonat eingesetzt werden. Als Alkalimetallhydroxid kann Natrium- oder Kaliumhydroxid und als Erdalkalimetall-hydroxid kann Calciumhydroxid verwendet werden. Die Umsetzung zum entsprechenden Salz erfolgt üblicherweise mit einer Mineralsäure wie mit Schwefelsäure, Salzsäure oder Phosphor-säure, vorzugsweise mit Salzsäure.

Das (1R,4S)- oder (1S,4R)-1-Amino-4-(hydroxymethyl)-2-cyclopenten hydrogen-D-tartrat und das (1R,4S)- oder (1S,4R)-1-Amino-4-(hydroxymethyl)-2-cyclopenten hydrogen-L-tartrat sind als nicht literaturbekannte Verbindungen ebenfalls Gegenstand der Erfindung.

Bevorzugt wird die chemische Racematspaltung mit D-(+)-Weinsäure wegen der höheren Leistung, technischen Leichtigkeit und effizienteren Racematspaltung durchgeführt.

Analog zum racemischen Aminoalkohol können selbstverständlich auch die optisch aktiven (1R,4S)- oder (1S,4R) -1-Amino-4-(hydroxymethyl)-2-cyclopentene mit D-(-)- oder L-(+)-Weinsäure zu den entsprechenden Tartraten umgesetzt werden.

Ein weiterer Bestandteil der vorliegenden Erfindung ist die Weiterumsetzung, die Acylierung, der (1R,4S)- oder (1S,4R)-1-Amino-4-(hydroxymethyl)-2-cyclopentene zu den (1R,4S)- oder (1S, 4R)-1-Amino-4-(hydroxymethyl)-2-cyclopentenderivaten der allgemeinen Formeln

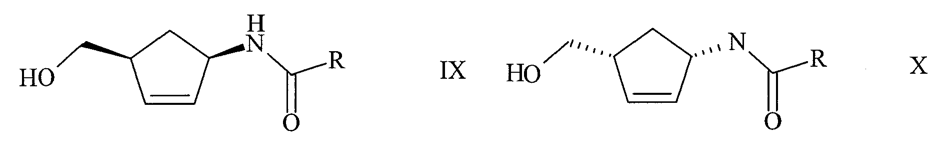

Dabei hat der Substituent R die gleiche Bedeutung wie im Cyclopentenderivat der allgemeinen Formel III.

Die Acylierung kann mit einem Carbonsäurehalogenid der allgemeinen Formel 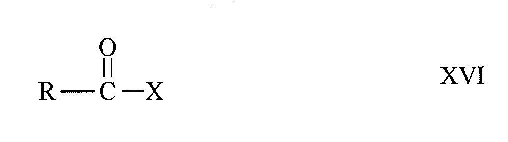 worin X ein Halogenatom bedeutet und R die genannte Bedeutung hat, oder mit einem Carbonsäureanhydrid der allgemeinen Formel 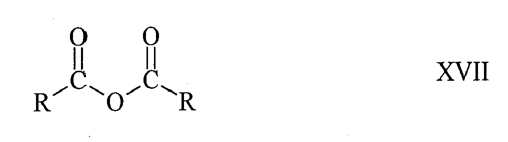 worin R die genannte Bedeutung hat, durchgeführt werden.
Als Halogenatom X können F, Cl, Br oder I verwendet werden. Vorzugsweise wird Cl oder F verwendet.

Beispiele für Carbonsäurehalogenide sind: Acetylchlorid, Chloracetylchlorid, Buttersäurechlorid, Isobuttersäurechlorid, Phenylessigsäurechlorid, Chlorameisensäurebenzylester (Cbz-Cl), Propionsäurechlorid, Benzoylchlorid, Chlorameisensäurealkylester oder tert-Butyloxycarbonylfluorid.

Beispiele für Carbonsäureanhydride sind: tert-Butoxycarbonylanhydrid, Buttersäureanhydrid, Essigsäureanhydrid oder Propionsäureanhydrid. Vorzugsweise wird die Acylierung mit einem Carbonsäureanhydrid, insbesondere mit tert-Butoxycarbonylanhydrid durchgeführt. Die Acylierung kann ohne Lösungsmittel oder mit einem aprotischen organischen Lösungsmittel durchgeführt werden. Zweckmässig wird die Acylierung in einem aprotischen organischen Lösungsmittel durchgeführt. Als aprotisches organisches Lösungsmittel sind beispielsweise Pyridin, Acetonitril, Dimethylformamid, Diisopropylether, Tetrahydrofuran, Toluol, Methylenchlorid, N-Methylpyrrolidon, Triethylamin, Chloroform, Essigsäureethylester und Essigsäureanhydrid bzw. Mischungen von diesen geeignet.

Zweckmässig wird die Acylierung bei einer Temperatur von -20 bis 100 °C, vorzugsweise von 0 bis 80 °C, durchgeführt.

Die erfindungsgemässe Weiterumsetzung vom (1R,4S)- oder (1S,4R)-1-Amino-4-(hydroxymethyl)-2-cyclopentenhydrogen-D- oder L-tartrat zum (1S,4R)- oder (1R,4S)-4-(2-amino-6-chlor-9-H-purin-9-yl)-2-cyclopenten-1-methanol, oder dessen Salzen, der Formeln 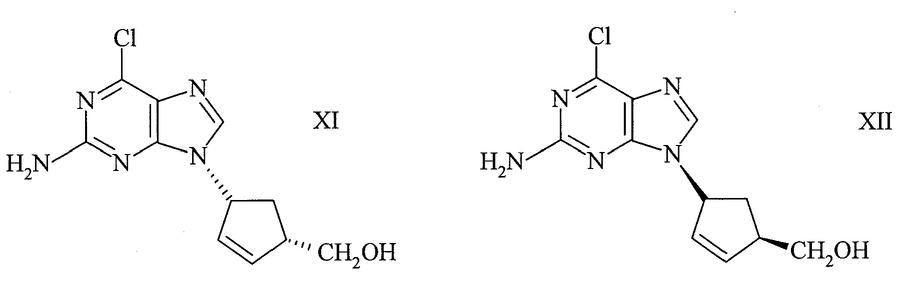 wird derart durchgeführt, dass man (1R,4S)- oder (1S,4R)-1-Ammo-4-(hydroxymethyl)-2-cyclopentenhydrogen-D- oder L-tartrat mit N-(2-amino-4,6-dichlorpyrimidin)-5-yl)formamid der Formel 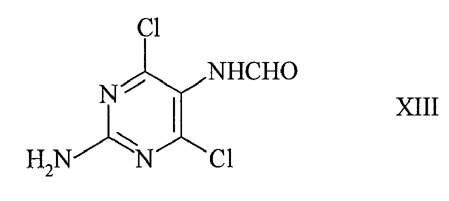 ins (1S,4R)- oder (1R,4S)-4-[(2-amino-6-chlor-5-formamido-4-pyrimidinyl)-amino]-2-cyclopenten-1-methanol der Formeln 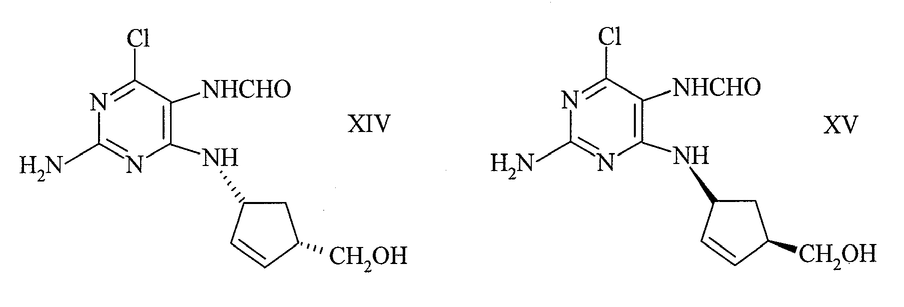 überführt und letzteres dann auf bekannte Weise zu den Verbindungen gemäss Formel XI und XII cyclisiert.

Das N-(2-amino-4,6-dichlorpyrimidin)-5-yl)formamid kann gemäss der WO 95/21 161 hergestellt werden.

Zweckmässig wird die Umsetzung in Gegenwart einer Base durchgeführt. Als Basen sind die gleichen geeignet, die wie zuvor beschrieben zur Freisetzung der (1R,4S)- oder (1S,4R)-1-Amino-4-(hydroxymethyl)-2-cyclopentene aus dem entsprechenden Tartrat eingesetzt werden.

Zweckmässig wird die Umsetzung in einem protischen Lösungsmittel durchgeführt. Als protisches Lösungsmittel können C₁₋₆-Alkohole wie Methanol, Ethanol, Propanol, Isopropanol, Butanol oder Isobutanol verwendet werden.

Dann wird das (1S,4R)- oder (1R,4S)-4-[(2-amino-6-chlor-5-formamido-4-pyrimidinyl)-amino]-2-cyclopenten-1-methanol der Formel XIV oder XV auf bekannte Weise gemäss der WO 95 / 21 161 ins Endprodukt gemäss Formel XI oder XII cyclisiert.

Üblicherweise wird die Cyclisierung gelöst in Trialkylorthoformiaten in Gegenwart von einer konzentrierten wässrigen Säure durchgeführt. Als Trialkylorthoformiate können beispielsweise Trimethyl- oder Triethylorthoformiat Verwendung finden. Als wässrige Säure kann beispielsweise Chlorwasserstoffsäure, Schwefelsäure oder Methansulfonsäure verwendet werden.

Ein weiterer Bestandteil der Erfindung ist das Gesamtverfahren zur Herstellung von (1S,4R)-4-(2-amino-6-chlor-9-H-purin-9-yl)-2-cyclopenten-1-methanol, bzw. dessen Salzen, der Formel XII ausgehend von (-)-2-Azabicyclo[2.2.1]hept-5-en-3-on oder (-)-Acyl-2-Azabicyclo-[2.2.1]hept-5-en-3-on der Formeln 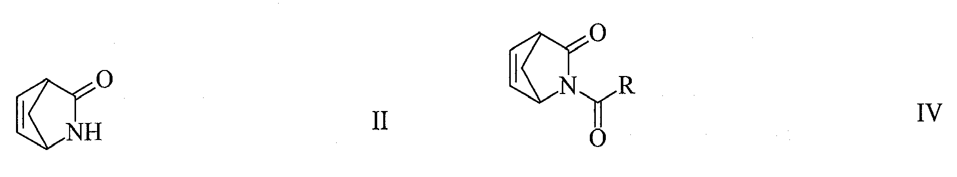 worin R die genannte Bedeutung hat, durch Reduktion mit einem Metallhydrid zu einem Aminoalkohol der Formel 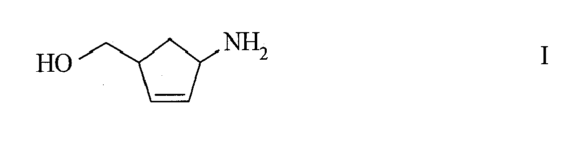 oder zu einem Cyclopentenderivat der allgemeinen Formel 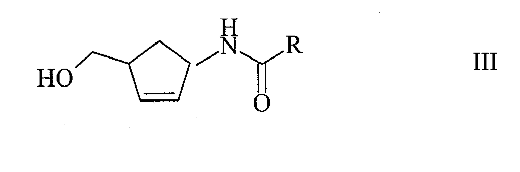 worin R die genannte Bedeutung hat, die dann in die entsprechenden Hydrohalogenidsalze überführt werden und dann mit N-(2-amino-4,6-dichlorpyrimidin)-5-yl)formamid der Formel 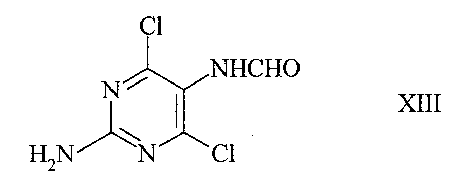 zum (1S,4R)-4-[(2-amino-6-chlor-5-formamido-4-pyrimidinyl)amino]-2-cyclopenten-1-methanol der Formel 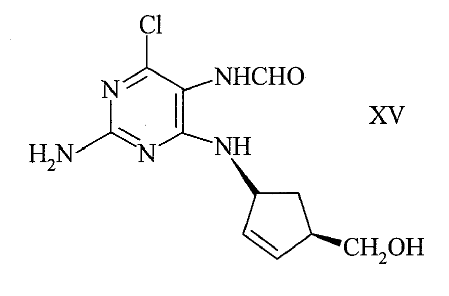 umgesetzt. Letzteres wird dann auf bekannte Weise zu der Verbindung der Formel 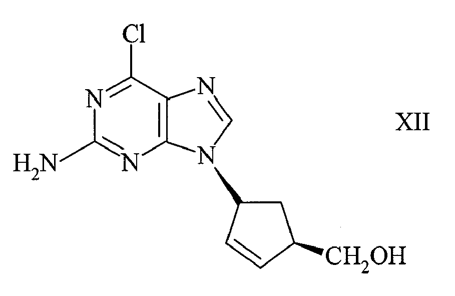 cyclisiert. Diese Verfahrensvariante hat den Vorteil, dass die dabei gebildeten Hydrohalogenidsalze als Rohgemisch in der Herstellung des Produktes der Formel XII verwendet werden können.

### Beispiele:

### Beispiel 1

### Reduktion von Acyl- oder unsubstituiertem 2-Azabicyclo[2.2.1]hept-5-en-3-on

### 1.1 Herstellung von cis-(±)-Acetyl-1-Amino-4-(hydroxymethyl)-2-cyclopenten in wasserfreien protischen organischen Lösungsmittel mittels Natriumborhydrid

280 g 2-Methyl-2-butanol (Amylalkohol) und 15,2 g Natriumborhydrid (0,4 mol) wurden in einem Sulfierkolben bei 20 °C vorgelegt. Zu dieser Suspension wurde ein Gemisch aus 907 g (±)-Acetyl-2-Azabicyclo[2.2.1]hept-5-en-3-on (0,6 mol) und 37,5 g Methanol (2 Aequivalente bezogen auf (±)-Acetyl-2-Azabicyclo[2.2.1]hept-5-en-3-on) innert 2 h bei 20 °C zudosiert. Anschliessend wurde das Reaktionsgemisch noch 3 h bei 20°C gerührt. Das Lösungsmittel wurde soweit wie möglich abdestilliert (40 °C). Zur Entfernung von Bor wurden 280 g Methanol und 27,2 g Ameisensäure zugegeben, das Gemisch wurde auf 25-30 °C erwärmt und das Azeotrop Methylborat / Methanol wurde bei dieser Temperatur abdestilliert (130 bis 80 mbar). Das ausgefallene Natriumformiat wurde abfiltriert, und das Filtrat eingeengt. Man erhielt 93,4 g Rohprodukt als klares viskoses Oel, Rohausbeute mit Gehalt: ca 84-85 %.

### 1.2 Herstellung von cis-(±)-1-Amino-4-(hydroxymethyl)-2-cyclopenten

Eine Suspension von (±)-2-Azabicyclo[2.2.1]hept-5-en-3-on (10,00 g, 91,6 mmol) und Lithiumborhydrid (4,00 g, 183,7 mmol) in trockenem Dioxan (100 ml) wurde unter Inertgasatmosphäre (Argon) 4 h bei 110 °C unter Rückflusstemperatur erhitzt. Nach dieser Zeit waren ca. 20 - 25 % des Eduktes zum Produkt umgesetzt (GC-Analyse mit internem Standard Benzophenon nach Aufarbeitung des Reaktionsgemisches; Aufarbeitung: 0,05 ml des Reaktionsgemisches wurden mit 0,1 ml 1M HCl gequencht und direkt im Anschluss mit 0,2 ml 1M NaOH basisch gestellt).
Der Strukturnachweis des Produktes erfolgte durch H-NMR, GC und GC-MS.

### 1.3 Herstellung von cis-(+)-1-Amino-4-(hydroxymethyl)-2-cyclopenten

In einem 25 ml Rundkolben wurden 1,0 g (9,2 mmol) (+)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃-Lösung bei pH 8 gepuffert. GC Analyse zeigte hier die Bildung des Produktes. Das gesamte Reaktionsgemisch wurde nun zur Trockene eingedampft und mittels Säulenchromatographie (Gradient: Hexan/Essigester/MeOH = 1:1:1 → MeOH) gereinigt. Auf diese Weise wurde cis-(+)-2-Azabicyclo[2.2.1]hept-5-en-3-on reisoliert und der entsprechende (+)-Aminoalkohol gewonnen.

### 1.4 Herstellung von cis-(-)-1-Amino-4-(hydroxymethyl)-2-cyclopenten

In einem 25 ml Rundkolben wurden 1,0 g (9,2 mmol) (-)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃-Lösung bei pH 8 gepufferet. GC Analyse zeigte hier die Bildung des Produktes in 18 % Ausbeute an. (GC-Standard ist Benzophenon). Das gesamte Reaktionsgemisch wurde nun zur Trockene eingedampft und mittels Säulenchromatographie (Gradient: Hexan/Essigester/MeOH = 1:1:1 → MeOH) gereinigt. Auf diese Weise wurde 0,43g (43 %) cis-(-)-2-Azabicyclo[2:2.1]hept-5-en-3-on reisoliert sowie 0,04g (4 %) entsprechender (-)-Aminoalkohol gewonnen.
Durch HPLC war nur das (-)-Enantiomer des Aminoalkohols detektierbar. Der ee des Produktes ist somit >98 %.

### 1.5 Herstellung von cis-(±)-1-Amino-4-(hydroxymethyl)-2-cyclopenten in einem Alkohol

In einem 100 ml Rundkolben mit magnetischer Rührung wurden 3,0 g (27,5 mmol) (±)-2-Azabicyclo[2.2.1]hept-5-en-3-on und 1,2 g (28,3 mmol) Lithiumborhydrid, unter Inertgasatmosphäre, in 35 g 2-Butanol vorgelegt und 3 h bei 60 °C gerührt.
GC-Analyse eines Musters (Aufarbeitung: 0,1 g Probe mit 0,2 ml 1M HCl sauergestellt, dann mit 0,1 ml gesättigter NaHCO₃ rasch basisch gestellt) zeigte nach dieser Zeit die Bildung des Produktes in 12 %iger Ausbeute an (GC-Standard ist Benzophenon).

### 1.6 Herstellung von cis-(±)-1-Amino-4-(hydroxymethyl)-2-cyclopenten in einem Alkohol/Ether-Gemisch

In einem 10 ml Rundkolben wurden unter Inertgasatmosphäre 0,5 g (4,6 mmol) (±)-2-Azabicyclo[2.2.1]hept-5-en-3-on und 0,59 g (18,4 mmol) Methanol in 7,5 ml Dioxan (abs.) vorgelegt. Dazu gab man 0,21 g (9,2 mmol) Lithiumborhydrid und heizte für 4 h bei 60°C. Dann kühlte man mit einem Eis/Wasser Bad auf 5°C und gab vorsichtig ca. 10 ml halbkonzentrierte HCl zum Reaktionsgemisch (heftige Reaktion, Gasentwicklung), wodurch eine gelblich klare Lösung entstand. Diese Lösung wurde direkt durch ein quantitatives ionenchromatografisches Verfahren analysiert. Sie enthielt 0,60 mmol (13,1%) cis-(±)-2-Azabicyclo[2.2.1]hept-5-en-3-on (bestimmt als HCl-Salz der entsprechenden Aminosäure, welche das saure Hydrolyseprodukt des (±)-2-Azabi-cyclo[2.2.1]hept-5-en-3-ons ist) und 3,06 mmol Produkt, entsprechend einer Ausbeute von 66,8%, Aminoalkohol.

### 1.7. Herstellung von cis-(±)-1-Amino-4-(hydroxymethyl)-2-cyclopenten in Gegenwart von Zusätzen wie Wasser oder verschiedenen Alkoholen

In einem 10 ml Rundkolben wurden 0,50 g (4,6 mmol) (±)-2-Azabicyclo[2.2.1]hept-5-en-3-on und 0,30 g (13,7 mmol) Lithiumborhydrid in 7,5 ml abs. Dioxan vorgelegt und auf 60 °C aufgeheizt. Bei dieser Temperatur wurde während 30 min. mittels Spritze X mmol des Zusatzes Y (Alkohol oder Wasser) zugetropft. Nun wurde 2 h bei 60 °C gerührt, abgekühlt auf ca. 20 °C und auf ca. 10 ml halbkonzentrierte HCl gegeben. Der Gehalt wurde dann direkt durch ein quantitatives ionenchromatografisches Verfahren bestimmt (vgl. Tabelle 1).

**Tabelle 1**

| Beispiel | Zusatz Y | X | X | (±)-2-Azabicyclo[2.2.1]-hept-5-en-3-on | Aminoalkohol |
|---|---|---|---|---|---|
| | | mmol | Aequivalente | Ausbeute % | |
| 1.7.1 | - | - | - | 15 | 52 |
| 1.7.2 | Wasser | 17,1 | 1,25 | 23,3 | 67,5 |
| 1.7.3 | Wasser | 34,3 | 2,5 | 32,3 | 58,3 |
| 1.7.4 | Methanol | 34,3 | 2,5 | 4,5 | 83,1 |
| 1.7.5 | Ethanol | 34,3 | 2,5 | 6,5 | 74,7 |
| 1.7.6 | Isopropanol | 34,3 | 2,5 | 28,1 | 52,3 |

### 1.8. Herstellung von cis-(±)-1-Amino-4-(hydroxymethyl)-2-cyclopenten mit verschiedenen Methanol-Mengen

Die Reaktion wurde unter den gleichen Bedingungen wie Beispiel 1.7 durchgeführt, jedoch mit der Ausnahme, dass anstatt des Zusatzes Y die Reaktion in verschiedenen Methanol-Konzentrationen durchgeführt wurde. Die Ergebnisse sind in Tabelle 2 zusammengefasst.

**Tabelle 2**

| Beispiel | Methanol | Methanol | (±)-2-Azabicyclo-[2.2.1] hept-5-en-3-on | Aminoalkohol |
|---|---|---|---|---|
| | mmol | Aequivalente | Ausbeute % | |
| 1.8.1 | 9,2 | 1 | 27,5 | 44,8 |
| 1.8.2 | 18,3 | 2 | 13,1 | 66,8 |
| 1.8.3 | 27,5 | 3 | 24,7 | 54,8 |
| 1.8.4 | 36,6 | 4 | 5,7 | 56,8 |
| 1.8.5 | 45,8 | 5 | 12,0 | 58,3 |
| 1.8.6 | 55,0 | 6 | 7,2 | 33,0 |

### 1.9 Herstellung von cis-(±)-1-Amino-4-(hydroxymethyl)-2-cyclopenten mit verschiedenen Lösungsmitteln

Die Reaktion wurde unter den gleichen Bedingungen wie Beispiel 1.7 durchgeführt, jedoch mit der Ausnahme, dass anstatt des Zusatzes Y 1,1 g Methanol zugetropft wurden und anstatt Dioxan als Lösungsmittel die Reaktion in verschiedenen Lösungsmitteln (7,5 ml) durchgeführt wurde und der Gehalt bestimmt wurde. Die Ergebnisse sind in Tabelle 3 zusammengefasst.

**Tabelle 3**

| Beispiel | Lösungsmittel X | (±)-2-Azabicyclo[2.2.1] hept-5-en-3-on | Aminoalkohol |
|---|---|---|---|
| | | Ausbeute % | |
| 1.9.1 | Dioxan | 13,6 | 79,8 |
| 1.9.2 | Diethylether | 10,8 | 68,6 |
| 1.9.3 | Tetrahydrofuran | 22,4 | 67,6 |
| 1.9.4 | Diisopropyl-Ether | 12,6 | 51,3 |
| 1.9.5 | tert-Butylmethyl-Ether | 10,0 | 71,3 |
| 1.9.6 | Mono-Glyme | 15,5 | 75,3 |
| 1.9.7 | Formaldehyddimethylacetal | 12,0 | 74,2 |

### 1.10 Herstellung von cis-(±)-1-Amino-4-(hydroxymethyl)-2-cyclopenten mit verschiedenen LiBH₄-Zugaben

Die Reaktion wurde unter den gleichen Bedingungen wie in Beispiel 1.7 durchgeführt, jedoch mit der Ausnahme, dass anstatt Zusatz Y 2,5 mol Methanol eingesetzt wurden und die Reaktion mit verschiedenen LiBH₄-Konzentrationen durchgeführt und der Gehalt bestimmt wurde. Die Ergebnisse sind in Tabelle 4 zusammengefasst.

**Tabelle 4**

| Beispiel | LiBH₄ | LiBH₄ | (±)-2-Azabicyclo[2.2.1] hept-5-en-3-on | Aminoalkohol |
|---|---|---|---|---|
| | mmol | Aequivalente | Ausbeute % | |
| 1.10.1 | 4,6 | 1 | 11,9 | 47,9 |
| 1.10.2 | 6,9 | 1,5 | 9,6 | 45,6 |
| 1.10.3 | 9,2 | 2 | 12,7 | 71,3 |
| 1.10.4 | 11,5 | 2,5 | 13,3 | 74,5 |
| 1.10.5 | 13,8 | 3 | 12,8 | 77,1 |
| 1.10.6 | 16,1 | 3,5 | 12,7 | 62,4 |

### 1.11 Herstellung von cis-(+)- oder (-)-1-Amino-4-(hydroxymethyl)-2-cyclopenten in Gegenwart verschiedener Akohole bzw. in Gegenwart von Wasser in verschiedenen Lösungsmitteln

In einem 10 ml Rundkolben mit magnetischer Rührung wurden 0,50 g (4,6 mmol) (+)- oder (-)-2-Azabicyclo[2.2.1]hept-5-en-3-on und 0,30 g (13,7 mmol) Lithiumborhydrid in 6 ml verschiedener Lösungsmittel vorgelegt und auf 60°C aufgeheizt. Bei dieser Temperatur wird während 30 min. mittels Spritze 34,3 mmol des Zusatzes Y zugetropft. Nun wurde 2h bei 60°C gerührt, abgekühlt auf ca. 20°C und auf ca. 10 ml halbkonzentrierte HCl gegeben.
Der Gehalt wird dann direkt durch ein quantitatives ionenchromatografisches Verfahren bestimmt (vgl. Tabelle 5). Der ee-Wert des Produktes wurde mittels HPLC ermittelt. Die Ergebnisse sind in Tabelle 5 zusammengefasst.

**Tabelle 5**

| | (-)-2-Azabicyclo[2.2.1] hept-5-en-3-on | (+)-2-Azabicyclo-[2.2.1]hept-5-en-3-on | | | Aminoalkohol | |
|---|---|---|---|---|---|---|
| Beispiel | ee-Wert | | Lösungsmittel | Zusatz Y | Ausbeute (IC) | ee-Wert (HPLC) |
| 1.11.1 | 98,0 | | Dioxan | Wasser | 64,3 | >99,0 |
| 1.11.2 | 98,0 | | Glyme | Wasser | 68,0 | >99,0 |
| 1.11.3 | 75,9 | | Dioxan | Wasser | 65,1 | 76,0 |
| 1.11.4 | 75,9 | | Glyme | Wasser | 63,5 | 75,6 |
| 1.11.5 | 50,2 | | Dioxan | Wasser | 74,8 | 51,4 |
| 1.11.6 | 51,6 | | Glyme | Wasser | 64,1 | 53,0 |
| 1.11.7 | 25,3 | | Dioxan | Wasser | 61,1 | 30,4 |
| 1.11.8 | 25,6 | | Glyme | Wasser | 61,0 | 29,6 |
| 1.11.9 | 98,0 | | Dioxan | Methanol | 83,1 | 98,2 |
| 1.11.10 | 98,0 | | Glyme | Methanol | 81,5 | 99,2 |
| 1.11.11 | 75,9 | | Dioxan | Methanol | 81,4 | 78,0 |
| 1.11.12 | 76,2 | | Glyme | Methanol | 79,9 | 78,6 |
| 1.11.13 | 50,4 | | Dioxan | Methanol | 81,3 | 54,4 |
| 1.11.14 | 51,5 | | Glyme | Methanol | 82,0 | 55,2 |
| 1.11.15 | 24,8 | | Dioxan | Methanol | 65,2 | 27,4 |
| 1.11.16 | 27,8 | | Glyme | Methanol | 81,7 | 32,2 |
| 1.11.17 | 98,0 | | Dioxan | Ethanol | 80,8 | 80,8 |
| 1.11.18 | 98,0 | | Glyme | Ethanol | 85,1 | 85,1 |
| 1.11.19 | 75,5 | | Dioxan | Ethanol | 85,3 | 78,2 |
| 1.11.20 | 75,6 | | Glyme | Ethanol | 83,6 | 78,4 |
| 1.11.21 | 50,7 | | Dioxan | Ethanol | 76,3 | 54,4 |
| 1.11.22 | 51,1 | | Glyme | Ethanol | 71,3 | 55,2 |
| 1.11.23 | 25,4 | | Dioxan | Ethanol | 73,0 | 28,6 |
| 1.11.24 | 25,5 | | Glyme | Ethanol | 75,0 | 28,6 |
| 1.11.25 | | 98,0 | Dioxan | Wasser | 62,0 | >99,0 |
| 1.11.26 | | 98,0 | Glyme | Wasser | 59,5 | >99,0 |
| 1.11.27 | | 51,3 | Dioxan | Wasser | 79,0 | 52,2 |
| 1.11.28 | | 49,0 | Glyme | Wasser | 61,3 | 52,0 |
| 1.11.29 | | 98,0 | Dioxan | Methanol | 77,2 | >99,0 |
| 1.11.30 | | 98,0 | Glyme | Methanol | 80,0 | >99,0 |
| 1.11.31 | | 49,0 | Dioxan | Methanol | 80,8 | 46,8 |
| 1.11.32 | | 49,5 | Glyme | Methanol | 80,9 | 48,8 |

### 1.12 Herstellung von cis-(±)-1-Amino-4-(hydroxymethyl)-2-cyclopenten mittels Natriumborhydrid in verschiedenen Alkoholen

Entsprechend zu Beispiel 1.7 wurde die Reaktion in verschiedenen Zusätzen (Alkohole oder Wasser) durchgeführt. Im Unterschied zu Beispiel 1.7 wurde jedoch Natriumborhydrid (0,51g; 13,7 mmol) als Reduktionsmittel eingesetzt. Die Ergebinsse sind in Tabelle 6 zusammengefasst.

**Tabelle 6**

| Beispiel | Zusatz Y | X | X | (+)-2-Azabicyclo[2.2.1] hept-5-en-3-on | Aminoalkohol |
|---|---|---|---|---|---|
| | | mmol | Aequivalente | Ausbeute % | |
| 1.12.1 | Wasser | 17,1 | 1,25 | 75,4 | 20,1 |
| 1.12.2 | Wasser | 34,3 | 2,5 | 71,9 | 26,7 |
| 1.12.3 | Methanol | 34,3 | 2,5 | 39,2 | 22,2 |
| 1.12.4 | Ethanol | 34,3 | 2,5 | 67,8 | 8,6 |
| 1.12.5 | - | - | - | 62,2 | 3,5 |

### 1.13 Herstellung von cis-(±)-1-Amino-4-(hydroxymethyl)-2-cyclopenten mit NaBH₃CN

In einem 100 ml Sulfierkolben mit mechanischer Rührung wurden 60 ml Dioxan und 8,6 g (137 mmol) Natriumcyanborhydrid sowie 11,9 g (137 mmol) Lithiumbromid über Nacht für 15 h bei 110°C rückflussiert. Dann wurde auf 60°C abgekühlt und eine Lösung von 5,0 g (45,8 mmol) (±)-2-Azabicyclo[2.2.1]hept-5-en-3-on mit 15 ml Methanol während 30 min. zugetropft. Die weisse Suspension wurde für 3 h bei 60°C gerührt, abgekühlt auf ca. 5°C und auf ca. 100 ml halbkonzentrierte HCl gegeben. Der Gehalt wurde dann direkt durch ein quantitatives ionenchromatografisches Verfahren bestimmt. Die Ausbeute an Aminoalkohol betrug ca. 4%.

### Beispiel 2

### Alkalische Hydrolyse von Acetyl-(±)-1-Amino-4-(hydroxymethyl)-2-cyclopenten

88,9 g racemisches Acetyl-1-amino-4-(hydroxymethyl)-2-cyclopenten (Gehalt 77,2 %) wurden suspendiert (teilweise gelöst) in 70 g Wasser. Dazu wurden 84 g 30 % NaOH (1,1 Aequivalente) zugegeben und die Lösung wurde 3 h rückflussiert. Laut DC war die Hydrolyse vollständig. Das entstandene Acetat wurde durch Elektrodialyse entfernt. Die erhaltene wässrige Lösung wurde eingeengt und getrocknet durch azeotrope Destillation mit Butanol. Der Rückstand wurde aufgenommen in Methanol zur Racematspaltung. Die Ausbeute der Hydrolyse zum (±)-1-Amino-4-(hydroxymethyl)-2-cyclopenten betrug 90 %.

### Beispiel 3: Herstellung von (1R,4S)- oder (1S,4R)-1-Amino-4-(hydroxymethyl)-2-cyclopenten

**3.1 Racematspaltung mittels Hydrolasen**
   **3.1.1 Herstellung von (1R,4S)-1-Amino-4-(hydroxymethyl)-2-cyclopenten mittels** Lipasen
      3.1.1.1 25 mM racemisches cis-1-Amino-4-(hydroxymethyl)2-cydopenten wurden mit 1000 Units Novozym 435 in 5 ml Dioxan bei Raumtemperatur suspendiert. 25 mM Methoxyessigsäureethylester wurden als Acetylierungsmittel zugesetzt. Die Bildung von N-Methoxyacetylaminoalkohol konnte mit DC eindeutig nachgewiesen werden. Der Umsatz betrug 50 % (nach Schätzung des DC). Dabei wurde das (1R,4S)-1-Amino-4-(hydroxymethyl)-2-cyclopenten gebildet.
      3.1.1.2 50 mM racemisches cis-1-Amino-4-(hydroxymethyl)-2-cyclopenten wurden mit 1000 Units (U) Novozym 435 in 5 ml Tetrahydrofuran suspendiert. Dieses wurde mit 50 mM NaOH und 50 mM Methoxyessigsäureethylester versetzt und bei 30 °C inkubiert. N-Methoxyacetylaminoalkohol konnte mit DC nachgewiesen werden. Der geschätzte Umsatz betrug 50 %. Dabei wurde das (1R,4S)-1-Amino-4-(hydroxymethyl)-2-cyclopenten gebildet.
      3.1.1.3 11 mg racemisches cis-1-Amino-4-(hydroxymethyl)-2-cyclopenten wurden mit 1 ml Methyl-tert.-butylether, 0,06 ml Tributyrin (Glycerintributyrat) und 20 U Novozym 435 (immob. Lipase aus Candida antarctica) bei Raumtemperatur gerührt. Nach 3 Tagen wurde laut HPLC enantiomerenreines (1R, 4S)-1-Amino-4-(hydroxymethyl)-2-cyclopenten in 43% Ausbeute erhalten.
      3.1.1.4 11 mg racemisches cis-1-Amino-4-(hydroxymethyl)-2-cyclopenten wurden mit 1 ml Methyl-tert.-butylether, 0,02 ml 6-Caprolacton und 20 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 87% ee in 49% Ausbeute erhalten (HPLC).
      3.1.1.5 100 mg racemisches cis-1-Amino-4-(hydroxymethyl)-2-cyclopenten wurden mit 1 ml Hexan, 0,3 ml Tributyrin und 20 U Novozym 435 (immob. Lipase aus Candida antarctica) bei Raumtemperatur gerührt. Nach 1 Woche wurde (1R, 4S)-1-Amino-4-(hydroxymethyl)-2-cyclopenten mit 77% ee in 28% Ausbeute erhalten (HPLC).
      3.1.1.6 100 mg racemisches cis-1-Amino-4-(hydroxymethyl)-2-cyclopenten wurden mit 1 ml tert.-Butanol, 0,3 ml Tributyrin und 20 U Novozym 435 (immob. Lipase aus Candida antarctica) bei 30°C gerührt. Nach 1 Woche wurde (1R, 4S)-1-Amino-4-(hydroxymethyl)-2-cyclopenten mit 78% ee in 15% Ausbeute erhalten (HPLC).
      3.1.1.7 11 mg racemisches cis-1-Amino-4-(hydroxymethyl)-2-cyclopenten wurden mit 1 ml Methyl-tert.-butylether, 0,2 mmol Capronsäuremethylester und 20 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 68% ee in 52% Ausbeute erhalten (HPLC).
      3.1.1.8 11 mg racemisches cis-1-Amino-4-(hydroxymethyl)-2-cyclopenten wurden mit 1 ml Methyl-tert.-butylether, 0,2 mmol Glykol-di-butyrat und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 89% ee in 31% Ausbeute erhalten (HPLC).
      3.1.1.9 11 mg racemisches cis-1-Amino-4-(hydroxymethyl)-2-cyclopenten wurden mit 1 ml Methyl-tert.-butylether, 0,2 mmol Fumarsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 86% ee in 26% Ausbeute erhalten (HPLC).
      3.1.1.10 11 mg racemisches cis-1-Amino-4-(hydroxymethyl)-2-cyclopenten wurden mit 1 ml Methyl-tert.-butylether, 0,2 mmol Malonsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 86% ee in 21 % Ausbeute erhalten (HPLC).
      3.1.1.11 11 mg racemisches cis-1-Amino-4-(hydroxymethyl)-2-cyclopenten wurden mit 1 ml Diisopropylether, 0,2 mmol Tributyrin und 40 U Novozym 435 (immob. Lipase aus Candida antarctica) bei Raumtemperatur gerührt. Nach 4 Tagen wurde enantiomerenreines (1R, 4S)-1-Amino-4-(hydroxymethyl)-2-cyclopenten in 15% Ausbeute erhalten (HPLC).
      3.1.1.12 11 mg racemisches cis-1-Amino-4-(hydroxymethyl)-2-cyclopenten wurden mit 1 ml Diisopropylether, 0,2 mmol Fumarsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 88% ee in 24% Ausbeute erhalten (HPLC).
      3.1.1.13 11 mg racemisches cis-1-Amino-4-(hydroxymethyl)-2-cyclopenten wurden mit 1 ml Diisopropylether, 0,2 mmol Malonsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 82% ee in 14% Ausbeute erhalten (HPLC).
      3.1.1.14 11 mg racemisches cis-1-Amino-4-(hydroxymethyl)-2-cyclopenten wurden mit 1 ml Diisopropylether, 0,2 mmol Diglykolsäure-diethylester und 40 U Novozym 435 (immob. Lipase aus Candida antarctica) bei Raumtemperatur gerührt. Nach 4 Tagen wurde (1R, 4S)-1-Amino-4-(hydroxymethyl)-2-cyclopenten mit 88% ee in 7% Ausbeute erhalten (HPLC).
      3.1.1.15 11 mg racemisches cis-1-Amino-4-(hydroxymethyl)-2-cyclopenten wurden mit 1 ml Dibutylether, 0,2 mmol Tributyrin und 40 U Novozym 435 (immobilisierte Lipase aus Candida antarctica) bei Raumtemperatur gerührt. Nach 4 Tagen wurde (1R, 4S)-1-Amino-4-(hydroxymethyl)-2-cyclopenten mit 95% ee in 13% Ausbeute erhalten (HPLC).
      3.1.1.16 11 mg racemisches cis-1-Amino-4-(hydroxymethyl)-2-cyclopenten wurden mit 1 ml Pyridin, 0,02 ml 2-Methoxyessigsäureethylester und 20 mg Lipase AK (Lipase aus Pseudomonas fluorescens) bei Raumtemperatur gerührt. Nach 4 Tagen wurde (1R, 4S)-1-Amino-4-(hydroxymethyl)-2-cyclopenten mit 84% ee in 18% Ausbeute erhalten (HPLC).
      3.1.1.17 11 mg racemisches cis-1-Amino-4-(hydroxymethyl)-2-cyclopenten wurden mit 1 ml Methyl-tert.-butylether, 0,2 mmol Cyanessigsäureethylester und 10 mg Lipase PS (Lipase aus Pseudomonas cepacia) bei Raumtemperatur gerührt. Nach 4 Tagen wurde (1R, 4S)-1-Amino-4-(hydroxymethyl)-2-cyclopenten mit 67% ee in 40% Ausbeute erhalten (HPLC).
      3.1.1.18 11 mg racemisches cis-1-Amino-4-(hydroxymethyl)-2-cyclopenten wurden mit 1 ml Methyl-tert.-butylether, 0,2 mmol Fumarsäurediethylester und 10 mg Lipase PS (Lipase aus Pseudomonas cepacia) bei Raumtemperatur gerührt. Nach 4 Tagen wurde (1R, 4S)-1-Amino-4-(hydroxymethyl)-2-cyclopenten mit 86% ee in 18% Ausbeute erhalten (HPLC).
   **3.1.2 Herstellung von (1R,4S)-1-Amino-4-(hydroxymethyl)-2-cyclopenten mittels Proteasen**
      3.1.2.1 11 mg racemisches cis-1-Amino-4-(hydroxymethyl)-2-cyclopenten wurden mit 1 ml 2-Methyl-2-butanol, 0,2 mmol Maleinsäurediethylester und 40 mg Alcalase (Protease aus Bacillus licheniformis) bei Raumtemperatur gerührt. Nach 4 Tagen wurde (1R, 4S)-1-Amino-4-(hydroxymethyl)-2-cyclopenten mit 28% ee in 39% Ausbeute erhalten (HPLC).
      3.1.2.2 11 mg racemisches cis-1-Amino-4-(hydroxymethyl)-2-cyclopenten wurden mit 1 ml 2-Methyl-2-butanol, 0,2 mmol Fumarsäurediethylester und 40 mg Savinase (Protease aus Bacillus sp.) bei Raumtemperatur gerührt. Nach 4 Tagen wurde (1R, 4S)-1-Amino-4-(hydroxymethyl)-2-cyclopenten mit 32% ee in 42% Ausbeute erhalten (HPLC).
      3.1.2.3 11 mg racemisches cis-1-Amino-4-(hydroxymethyl)-2-cyclopenten wurden mit 1 ml 2-Methyl-2-butanol, 0,06 ml Tributyrin und 20 mg Savinase (Protease aus Bacillus sp.) bei Raumtemperatur gerührt. Nach 4 Tagen wurde (1R, 4S)-1-Amino-4-(hydroxymethyl)-2-cyclopenten mit 22% ee in 39% Ausbeute erhalten (HPLC).
      3.1.2.4 11 mg racemisches cis-1-Amino-4-(hydroxymethyl)-2-cyclopenten wurden mit 1 ml 2-Methyl-2-butanol, 0,06 ml Tributyrin und 20 mg Subtilisin (Protease aus Bacillus licheniformis) bei Raumtemperatur gerührt. Nach 4 Tagen wurde (1R, 4S)-1-Amino-4-(hydroxymethyl)-2-cyclopenten mit 23% ee in 36% Ausbeute erhalten (HPLC).
   **3.1.3 Herstellung von (1S,4R)-1-Amino-4-(hydroxymethyl)-2-cyclopenten mittels Proteasen**
      3.1.3.1 11 mg racemisches cis-1-Amino-4-(hydroxymethyl)-2-cyclopenten wurden mit 1 ml Hexan, 0,06 ml Tributyrin und 120 U Savinase (Protease aus Bacillus sp.) bei Raumtemperatur gerührt. Nach 3 - 6 Tagen wurde (1S, 4R)-1-Amino-4-(hydroxymethyl)-2-cyclopenten mit 44% ee in 46% Ausbeute erhalten (HPLC).
      3.1.3.2 11 mg racemisches cis-1-Amino-4-(hydroxymethyl)-2-cyclopenten wurden mit 1 ml Hexan, 0,06 ml Tributyrin und 20 mg Alcalase (Protease aus Bacillus licheniformis) bei Raumtemperatur gerührt. Nach 3-6 Tagen wurde (1S, 4R)-1-Amino-4-(hydroxymethyl)-2-cyclopenten mit 44% ee in 35% Ausbeute erhalten (HPLC).
   **3.1.4 Herstellung von (1S,4R)-1-Amino-4-(hydroxymethyl)-2-cyclopenten mittels Lipasen**
      3.1.4.1 11 mg racemisches cis-1-Amino-4-(hydroxymethyl)-2-cyclopenten wurden mit 1 ml 2-Methyl-2-butanol, 0,03 ml Ethylbutyrat und 20 mg Newlase F (Lipase aus Rhizopus niveus) bei Raumtemperatur gerührt. Nach 1 Woche wurde (1S, 4R)-1-Amino-4-(hydroxymethyl)-2-cyclopenten mit 39% ee in 37% Ausbeute erhalten (HPLC).
      3.1.4.2 11 mg racemisches cis-1-Amino-4-(hydroxymethyl)-2-cyclopenten wurden mit 1 ml Pyridin, 0,06 ml Tributyrin und 20 mg Lipase AK (Lipase aus Pseudomonas fluorescens) bei Raumtemperatur gerührt. Nach 1 Woche wurde (1S, 4R)-1-Amino-4-(hydroxymethyl)-2-cyclopenten mit 30% ee in 10% Ausbeute erhalten (HPLC).
      3.1.4.3 11 mg racemisches cis-1-Amino-4-(hydroxymethyl)-2-cyclopenten wurden mit 1 ml 2-Methyl-2-butanol, 0,06 ml Tributyrin und 20 mg Lipase AY (Lipase aus Candida rugosa) bei Raumtemperatur gerührt. Nach 1 Woche wurde (1S, 4R)-1-Amino-4-(hydroxymethyl)-2-cyclopenten mit 32% ee in 13% Ausbeute erhalten (HPLC).
      3.1.4.4 11 mg racemisches cis-1-Amino-4-(hydroxymethyl)-2-cyclopenten wurden mit 1 ml Methyl-t.-butylether, 0,06 ml Tributyrin und 20 mg Lipase PS-CI (immobilisierte Lipase aus Pseudomonas cepacia) bei Raumtemperatur gerührt. Nach 1 Woche wurde (1S, 4R)-1-Amino-4-(hydroxymethyl)-2-cyclopenten mit 29% ee in 16% Ausbeute erhalten (HPLC).
      3.1.4.5 11 mg racemisches cis-1-Amino-4-(hydroxymethyl)-2-cyclopenten wurden mit 1 ml Methyl-t.-butylether, 0,06 ml Tributyrin und 20 mg Lipase PS (Lipase aus Pseudomonas cepacia) bei Raumtemperatur gerührt. Nach 1 Woche wurde (1S, 4R)-1-Amino-4-(hydroxymethyl)-2-cyclopenten mit 24% ee in 22% Ausbeute erhalten (HPLC).
**3.2 Racematspaltung mit D-(-)-Weinsäure**
   3.2.1 Ein Gemisch aus 8 g (70,6 mmol) racemisches 1-Amino-4-(hydroxymethyl)-2-cyclopenten und 10,6 g (70,6 mmol) D-(-)-Weinsäure in 186 g Methanol wurde bei Rückflusstemperatur gelöst. Anschliessend wurde in 2 h auf 20 °C gekühlt. Bei 43 °C wurden Impfkristalle des reinen (1R,4S)-1-Amino-4-(hydroxymethyl)-2-cyclopenten hydrogen-D-tartrat zugegeben. Das kristallisierte Produkt wurde abfiltriert und getrocknet. Ausbeute : 8,49 g (45,6 % bezogen auf racemisches Edukt) (1R,4S)-1-Amino-4-(hydroxymethyl)-2-cyclopenten hydrogen-D-tartrat, ee-Wert : 91,1 %. Zur Reinigung wurden 8,49 g (32,25 mmol) des Hydrogentartrats in 30 ml Methanol suspendiert und 2 Äquivalente 30 % Natriummethylat zugegeben. Das Natriumtartrat wurde abfiltiert und das Methanol abdestilliert.
      Der Rückstand wurde in 35 ml Pentanol aufgenommen. Darauf wurden bei 55 °C 1,5 g HCl eingeleitet, die Lösung wurde langsam abgekühlt. Bei 40°C wurde die Lösung mit (1R,4S)-1-Amino-4-(hydroxymethyl)-2-cyclopenten-hydrochlorid angeimpft.
      Anschliessend wurden 45 ml Aceton zudosiert, die Suspension langsam auf 0 °C abgekühlt, filtriert und der Rückstand getrocknet.
      Es wurden 3,91 g (1R,4S)-1-Amino-4-(hydroxymethyl)-2-cyclopenten hydrochlorid mit einem ee-Wert von > 98 % erhalten, entsprechend einer Ausbeute bezogen auf eingesetztes racemisches (1R,4S)-1-Amino-4-(hydroxymethyl)-2-cyclopenten von 37 %.
   3.2.2 Ein Gemisch aus 64 g racemisches 1-Amino-4-(hydroxymethyl)-2-cyclopenten (0,5 mol) und 75,2 g D-(-)-Weinsäure in 1330 g Methanol wurde bei Rückflusstemperatur gelöst und anschliessend in 2 h auf 20 °C gekühlt. Bei 43 °C wurden Impfkristalle des reinen 1R,4S-Enantiomers zugegeben. Das kristallisierte Produkt wurde abfiltriert und getrocknet. Ausbeute : 63,2 g (48,0 % bezogen auf racemisches 1-Amino-4-(hydroxymethyl)-2-cyclopenten) 1R,4S-(1-Amino-4-(hydroxymethyl)-2-cyclopenten-hydrogentartrat, ee-Wert : 91,1 %. Der ee-Wert in der Mutterlauge betrug 76,0 %.
   **3.2.3 Umkristallisation von 1R,4S-(4-Amino-2-cyclopenten-1-yl)-methanol-D-hydrogentartrat**
      61,84 g 1R,4S-(4-Amino-2-cyclopenten-1-yl)methanol-D-hydrogentartrat (0,235 mol, ee-Wert 91,1 %) wurden gelöst in 752 g Methanol unter Rückfluss. Die Lösung wurde abgekühlt auf 20 °C innert 90 min., anschliessend wurde das Produkt abfiltriert und mit 64 g kaltem Methanol nachgewaschen. Nach Trocknung wurde 54,56 g 1R,4S-(4-Amino-2-cyclopenten-1-yl)methanol-D-hydrogentartrat erhalten, ee-Wert 99,4 % (Ausbeute 88,2 %, 42,3 % bezüglich racemischem 1-Amino-4-(hydroxymethyl)-2-cyclopenten). Dieses wurde tel quel in die Chlorpurinsynthese eingesetzt.
   3.2.4 Analog zu Beispiel 3.2.2, aber mit 223 g Methanol und Animpfen bei 50 °C wurde die Racemattrennung durchgeführt. Die Ausbeute betrug 7,98 g (42,9 % bezogen auf eingesetztes racemisches (1R,4S)-1-Amino-4-(hydroxymethyl)-2-cyclopenten).
**3.3 Racematspaltung mit L-(+)-Weinsäure**
   3.3.1 Ein Gemisch aus 8 g (70,6 mmol) racemischem 1-Amino-4-(hydroxymethyl)-2-cyclopenten und 10,6 g (70,6 mmol) L-(+)-Weinsäure in 186 g Methanol wurde bei Rückflusstemperatur gelöst. Anschliessend wurde in 2 h auf 20 °C gekühlt. Bei 43 °C wurden Impfkristalle des reinen (1S,4R)-1-Amino-4-(hydroxymethyl)2-cyclopenten hydrogen-L-tartrat zugegeben. Das kristallisierte (1S,4R)-1-Amino-4-(hydroxymethyl)-2-cyclopenten hydrogen-L-tartrat wurde abfiltriert und getrocknet. (ee-Wert : 91,1 %) Zu der Mutterlauge wurden 14 g 30 % methanolisches Natriummethylat zugegeben, darauf das Methanol eingeengt. Der Rückstand wurde in 35 ml Isobutanol aufgenommen und das unlösliche Natriumtartrat abfiltriert. In das Filtrat wurden bei 55 °C 2 g gasförmige HCl eingeleitet. Man gab dann 38 ml Aceton hinzu und liess innerhalb von 1 h auf 10 °C abkühlen. Nach 1 h wurde das (1R,4S)-1-Amino-4-(hydroxymethyl)-2-cyclopenten-hydrochlorid abgenutscht und mit 8 ml Aceton nachgewaschen. Nach dem Trocknen im Vakuum erhielt man das (1R,4S)-1-Amino-4-(hydroxymethyl)-2-cyclopenten-hydrochlorid in einer Ausbeute von 34 g 31,6 % bezogen auf racemisches 1-Amino-4-(hydroxymethyl)-2-cyclopenten mit einem ee-Wert von > 98 % .

### Beispiel 4: Herstellung von (1R,4S)-Amino-4-(hydroxymethyl)-2-cyclopentenhydrochlorid

### 4.1 Reduktion von (-)-2-Azabicyclo[2.2.1]hept-5-en-3-on

In einen mit N₂ inertisierten 21- Autoklaven (V4A) wurden 61,4 g Natriumborhydrid 97,5% (1,623 Mol), 70,2 g Lithiumchlorid 98,5% (1,656 Mol), 13,2 g Celite sowie 1410 g Tetrahydrofuran eingefüllt. Der Autoklav wurde verschlossen, es wurde auf eine Innentemperatur von 130 °C aufgeheizt und 4,5 Stunden bei dieser Temperatur gerührt (max. 8,0 bar).

Nach Abkühlen auf ca. 60 °C wurden die in Tetrahydrofuran unlöslichen Natriumsalze (NaCl, NaBH₄) abfiltriert. Diese wurden mit 353 g Tetrahydrofuran gewaschen und die vereinigten Filtrate wurden in einem 1 1-Glasrührwerk durch Destillation bei Normaldruck auf ca. die Hälfte eingeengt (Destillat 1: ca. 710 g Tetrahydrofuran). Anschliessend wurde durch weitere Destillation, im Wechsel mit portionsweiser Zugabe von insgesamt 936 g Dioxan der Lösungsmitteltausch vollendet (Destillat 2: ca. 1289 g Tetrahydrofuran/Dioxan).
Zur auf ca. 60 °C gekühlten LiBH₄-Suspension wurden 56,7 g (-)-2-Azabicyclo[2.2.1]hept-5-en-3-on (97,5%) addiert.
Bei ca. 60 °C beginnend wurden in exakt einer Stunde 132,5 g Methanol so zudosiert, dass ein Temperaturbereich von 58 - 62 °C eingehalten wurde. Es wurde eine weitere Stunde bei 60 °C nachreagieren gelassen. Anschliessend wurden weitere 397,0 g Methanol addiert (Probe enthält eine analytische Ausbeute von 70,5%) und der Rührwerksinhalt wurde auf 0 °C abgekühlt. Bei dieser Temperatur wurden 90,0 g HCl in die Reaktionsmischung eingeleitet (leicht exotherm) und es wurde eine weitere Stunde bei ca 0 °C gerührt. Durch Destillation bei Normaldruck (bis zu einer Kopftemperatur von 75 °C) wurden die Leichtsieder (Methanol, Borester) und ca. 70% des Dioxans entfernt (Destillat 3: ca. 1093 g). Anschliessend wurde durch Vakuumdestillation (ca. 30 mbar), im Wechsel mit portionsweiser Zugabe von insgesamt 282 g 1-Pentanol der Lösungsmitteltausch vollendet (Destillat 4: ca. 240 g Dioxan / Pentanol). Nach Zugabe weiterer 302 g 1-Pentanol wurde 1 Stunde bei 50 °C gerührt und die ausgefallenen Salze, ca. 39 g Feuchtgewicht, wurden abfiltriert und mit 200 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 236 g Aceton zudosiert und die Reaktionsmischung wurde mit einigen Kristallen (1R,4S)-Amino-4-(hydroxymethyl)-2-cyclopenten angeimpft. Innerhalb einer Stunde wurde auf 5 °C abgekühlt und zur Vollendung der Kristallisation wurde weitere 6 h bei 5 °C gerührt.

Das Kristallisat wurde abfiltriert, mit 63 g Aceton gewaschen und bei max. 50 °C im Vakuumtrockenschrank (10 mbar) getrocknet. Man erhielt 83,5 g Rohprodukt * (Gehalt: 56,5%).
Dies entsprach einer Ausbeute von 61,4 % bzgl. eingesetztem (-)-2-Azabicyclo-[2.2.1]hept-5-en-3-on.

### 4.2 Reduktion von (±)-2-Azabicyclo[2.2.1]hept-5-en-3-on

In einen mit N₂ inertisierten 21- Autoklaven (V4A) wurden 41,56 g Natriumborhydrid 97,5% (1,071 Mol), 51,48 g Lithiumchlorid 98,5% (1,196 Mol), 9,30 g Celite sowie 955,0 g Tetrahydrofuran eingefüllt. Der Autoklav wurde verschlossen, es wurde auf eine Innentemperatur von 130 °C aufgeheizt und 6 Stunden bei dieser Temperatur gerührt (max. 6,3 bar).
Nach Abkühlen auf ca. 60 °C wurden die in Tetrahydrofuran unlöslichen Natriumsalze (NaCl, NaBH₄) abfiltriert. Diese wurden mit 239,0 g Tetrahydrofuran gewaschen und die vereinigten Filtrate wurden in einem 1 1-Glasrührwerk durch Destillation bei Normaldruck auf ca die Hälfte eingeengt (Destillat 1: ca. 590 g THF).
Anschliessend wurde durch weitere Destillation, im Wechsel mit portionsweiser Zugabe von insgesamt 661,0 g Dioxan der Lösungsmitteltausch vollendet (Destillat 2: ca. 685 g Tetrahydrofuran/Dioxan). Zur auf ca. 60 °C gekühlten LiBH₄-Suspension wurden 36,0 g 2-Azabicyclo[2.2.1]hept-5-en-3-on (97,5%) addiert. Bei ca. 60 °C beginnend wurden in exakt einer Stunde 77,6 g Methanol so zudosiert, dass ein Temperaturbereich von 58 - 62 °C eingehalten wurde. Es wurde eine weitere Stunde bei 60 °C nachreagieren gelassen. Anschliessend wurden weitere 233,0 g Methanol addiert und der Rührwerksinhalt wurde auf 0 °C abgekühlt. Bei dieser Temperatur wurden 52,9 g HCl in die Reaktionsmischung eingeleitet (leicht exotherm) und es wurde eine weitere Stunde bei ca 0 °C gerührt.
Durch Destillation bei Normaldruck (bis zu einer Kopftemperatur von 75 °C) wurden die Leichtsieder (Methanol, Borester) und ca. 70% des Dioxans entfernt (Destillat 3: ca. 700 g).

Anschliessend wurde durch Vakuumdestillation (ca. 30 mbar), im Wechsel mit portionsweiser Zugabe von insgesamt 169,4 g 1-Pentanol der Lösungsmitteltausch vollendet (Destillat 4: ca. 183 g Dioxan / Pentanol). Nach Zugabe weiterer 127,1 g 1-Pentanol wurde 1 Stunde bei 50 °C gerührt und die ausgefallenen Salze, ca.41 g Feuchtgewicht, wurden abfiltriert und mit 63,5 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 238,0 g Aceton zudosiert und die Reaktionsmischung wurde mit einigen Kristallen Aminoalkohol-Hydrochloridsalz angeimpft. Innerhalb einer Stunde wurde auf 5 °C abgekühlt und zur Vollendung der Kristallisation wurde weitere 6 Stunden bei 5 °C gerührt.
Das Kristallisat wurde abfiltriert, mit 61,0 g Aceton gewaschen und bei max. 50 °C im Vakuumtrockenschrank (10 mbar) getrocknet. Man erhielt 50,0 g Rohprodukt (Gehalt: ca. 50% Aminoalkohol-Hydrochloridsalz). Dies entsprach einer Ausbeute von 52,0 % bzgl. eingesetztem 2-Azabicyclo[2.2.1]hept-5-en-3-on.

### Beispiel 5: Herstellung von acylierten Aminoalkoholen

### 5.1 Herstellung von (1R,4S)-N-BOC-1-amino-4-(hydroxymethyl)-2-cyclopenten (BOC = tert-Butoxycarbonyl)

75 g einer Lösung von (1R,4S)-1-Amino-4-hydroxymethyl-2-cyclopenten wurde mit 30 %igen NaOH auf pH 8 gestellt und mit 6 g NaHCO₃ versetzt. Das Gemisch wurde auf 52 °C erwärmt. Unter guter Rührung wurde dazu 60 ml Diisopropylether zugegeben und dann während 2 h eine Lösung von 11,12 g BOC-Anhydrid in 18,2 ml Diisopropylether zudosiert. Die Mischung wurde über Celite filtriert und die Phasen getrennt. Die Wasserphase wurde mit 65 ml Diisopropylether extrahiert. Die vereinigten organischen Phasen wurden mit 45 ml Wasser gewaschen, dann auf 37,5 g eingedampft und auf 50 °C erwärmt. Zu der Lösung wurde 31 ml n-Hexan zugetropft. Nach langsamer Kühlung auf 0 °C (2 h), wurde die Titelverbindung filtriert, mit 12 ml n-Hexan / Diisopropylether 1/1 gewaschen und getrocknet. Man erhielt 6,75 g Produkt. Die Ausbeute betrug 71 %.

### 5.2 Herstellung von (1R,4S)-N-Acetyl-1-amino-4-(hydroxymethyl)-2-cyclopenten

25 g (1R,4S)-1-Amino-4-(hydroxymethyl)-2-cyclopenten-hydrochlorid wurden in 182 ml Essigsäureanhydrid gelöst und bei 0 °C mit einer Lösung von 18,25 g Triethylamin in 60 ml Essigsäureanhydrid versetzt. Die Mischung wurde bei 80 °C 3 h gerührt, dann auf Raumtemperatur abgekühlt. Das Triethylaminhydrochlorid wurde abfiltriert und mit 120 ml n-Hexan gewaschen. Das Filtrat wurde eingedampft. Der Rückstand wurde mit 300 ml Toluol versetzt und bei Raumtemperatur in Gegenwart von 5,2 g Aktivkohle und 13 g Celite 20 Min. gerührt. Nach Filtration und Waschen des Filterkuchens (3mal 40 ml Toluol), wurde das Lösungsmittel vollständig eingeengt. Der Rückstand wurde mit 180 ml Methanol und 15,5 g K₂CO₃ versetzt und bei Raumtemperatur 10 h gerührt. Die Suspension wurde abfiltriert und das Filtrat eingedampft. Der Rückstand wurde in 750 ml Isopropylacetat suspendiert und in Gegenwart von 0,5 g Aktivkohle auf Rückfluss 1,5 h abgekocht. Nach der Aktivkohle-Filtration (70 - 80 °C) wurde das Filtrat über Nacht bei 0 °C gekühlt. Die Titelverbindung wurde filtriert, mit 80 ml kaltem Isopropylacetat gewaschen und unter Vakuum getrocknet. Man erhielt 17,2 g Produkt. Die Ausbeute betrug 66 %.

### 5.3 Herstellung von (1R,4S)-N-Butyryl-1-amino-4-(hydroxymethyl)-2-cyclopenten

34,7 g (1R,4S)-1-Amino-4-hydroxymethyl-2-cyclopenten-hydrochlorid und 2 g N,N-4-Dimethylaminopyridin wurden in 600 ml Methylenchlorid gelöst. Die Lösung wurde auf 0 °C gekühlt. Dann wurden 52 g Triethylamin zugetropft (5 Min.). Das Gemisch wurde noch 30 Min. weitergerührt. Zu der Mischung wurde bei 0 °C eine Lösung von 35,2 g Butyrylchlorid in 60 ml Methylenchlorid während 1 h zudosiert. Das Gemisch wurde noch zwischen 0 bis 20 °C 1,5 h weitergerührt, dann mit 600 ml Wasser versetzt.

Nach Phasentrennung wurde die Wasserphase mit 600 ml Methylenchlorid extrahiert. Die vereinigten organischen Phasen wurden 3mal mit je 500 ml 10 %iger NaOH gewaschen, dann vollständig eingedampft. Der getrocknete Feststoff wurde in 120 ml Methanol gelöst. Die Lösung wurde mit 5 g K₂CO₃ versetzt und bei Raumtemperatur 2 h weitergerührt. Die anorganischen Salze wurden abfiltriert und mit 20 ml Methanol gespült. Das Filtrat wurde mit 2N HCl neutralisiert. Die Suspension wurde abfiltriert und der Filterkuchen mit 20 ml Methanol gewaschen. Das Filtrat wurde vollständig eingedampft. Der feste Rückstand wurde getrocknet und in 150 ml Toluol kristallisiert. Man erhielt 28,5 g Titelverbindung. Die Ausbeute betrug 67 %.

### Beispiel 6: Herstellung von [4(R)-(2-Amino-6-chloropurin-9-yl)cyclopent-2-en-1(S)-yl]methanol

### 6.1 Herstellung von [4(R)-(2-Amino-6-chloropurin-9-yl)cyclopent-2-en-1(S)-yl]methanol ausgehend von 1R,4S-(4-Amino-2-cyclopenten-1-yl)methanol-D-hydrogentartrat

47,4 g 1R,4S-(4-Amino-2-cyclopenten-1-yl)methanol-D-hydrogentartrat (0,18 mol, ee > 98 %) in 200 ml Ethanol wurde vorgelegt. Bei Raumtemperatur wurden 54,6 g NaHCO₃ (0,65 mol) und 37,3 g (0,18 mol) N-(2-Amino-4,6-dichlor-4-pyrimidyl)formamid zugegeben, 9 h unter Rückfluss gekocht und dann auf Raumtemperatur gekühlt. Die Salze wurden abfiltriert und mit 50 ml Ethanol nachgewaschen. Das Filtrat wurde im Rotavapor auf 280 g aufkonzentriert. In der erhaltenen Lösung wurden 18,4 g HCl Gas bei T < 25 °C eingeleitet, dann wurden 95,5 g (0,9 mol) Trimethylorthoformiat zugegeben und das Ganze auf 40 °C aufgeheizt (10 min). Bei dieser Temperatur wurde mit Chlorpurin Hydrochlorid angeimpft. Nach 2 h bei 42 °C kristallisierte das Produkt aus. Die Suspension wurde auf 15 °C gekühlt. Das Produkt wurde filtriert und 3mal mit 50 ml Ethanol nachgewaschen, anschliessend bei 50 °C in Vakuum getrocknet. Die Ausbeute betrug 41,9 g (75.8 %). Beiges Pulver, Gehalt (HPLC) : 95.0 %.

### 6.2 Herstellung von [4(R)-(2-Amino-6-chloropurin-9-yl)cyclopent-2-en-1(S)-yl]-methanol ausgehend von (-)-2-Azabicyclo[2.2.1]hept-5-en-3-on

In einen mit N₂ inertisierten 21- Autoklaven (V4A) wurden 61,4 g Natriumborhydrid 97,5% (1,623 Mol), 70,2 g Lithiumchlorid 98,5% (1,656 Mol), 13,2 g Celite sowie 1410 g Tetrahydrofuran eingefüllt. Der Autoklav wurde verschlossen, es wurde auf eine Innentemperatur von 130 °C aufgeheizt und 4,5 Stunden bei dieser Temperatur gerührt (max. 8,0 bar). Nach Abkühlen auf ca. 60 °C wurden die in Tetrahydrofuran unlöslichen Natriumsalze (NaCl, NaBH₄) abfiltriert. Diese wurden mit 353 g Tetrahydrofuran gewaschen und die vereinigten Filtrate wurden in einem 11-Glasrührwerk durch Destillation bei Normaldruck auf ca die Hälfte eingeengt (Destillat 1: ca. 710 g Tetrahydrofuran). Anschliessend wurde durch weitere Destillation, im Wechsel mit portionsweiser Zugabe von insgesamt 936 g Dioxan der Lösungsmitteltausch vollendet (Destillat 2: ca. 1289 g Tetrahydrofuran/Dioxan). Zur auf ca. 60 °C gekühlten LiBH₄-Suspension wurden 56,7 g (-)-2-Azabicyclo[2.2.1]hept-5-en-3-on (97,5% / 0,507 Mol) addiert.
Bei ca. 60 °C beginnend wurden in exakt einer Stunde 132,5 g Methanol so zudosiert, dass ein Temperaturbereich von 58 - 62 °C eingehalten wurde. Es wurde eine weitere Stunde bei 60 °C nachreagieren gelassen. Anschliessend wurden weitere 397,0 g Methanol addiert (Probe enthält eine analytische Ausbeute von 70,5%) und der Rührwerksinhalt wurde auf 0 °C abgekühlt. Bei dieser Temperatur wurden 90,0 g HCl in die Reaktionsmischung eingeleitet (leicht exotherm) und es wurde eine weitere Stunde bei ca 0 °C gerührt. Die Lösung wurde am Rotavapor bei 50° C unter Vakuum eingeengt, 200 ml Methanol wurden zugesetzt und das Methanol wurde wieder entfernt (absaugen des Methylborates). Vorgang wurde mit weiteren 200 ml Methanol wiederholt. Das erhaltene Oel (253,4 g enthalten 3,16 % Aminoalkohol dies entsprach 0,360 Mol) wurde mit 250 ml Ethanol versetzt und in einem 11-Doppelmantelrührgefäss gegeben. Bei Raumtemperatur 72,6 g NaHCO₃ (0,86 Mol) und 74,6 g (0,360 Mol) N-(2-Amino-4,6-dichlor-4-pyrimidyl)formamid zugeben, 9 h wurde rückflussiert, auf Raumtemperatur gekühlt, Salze abfiltriert und mit 100 ml Ethanol nachwaschen.

Das Filtrat im Rotavapor wurde auf 560 g aufkonzentriert. In die erhaltene Lösung wurden 63,4 g HCl-Gas bei T < 25 °C eingeleitet, dann 191,0 g (1,80 Mol) Trimethylorthoformiat zugeben, auf 40 °C aufgeheizt (10 min). Bei dieser Temperatur mit Chlorpurin Hydrochlorid angeimpft, 2 h bei 42 °C wurde kristallisiert gelassen. Die Suspension wurde auf 15 °C gekühlt. Das Produkt wurde filtriert und mit 3-mal 50 ml Ethanol nachgewaschen, anschliessend bei 50 °C i.V. getrocknet. Die Ausbeute betrug 66,0 g (59,7 %). Beiges Pulver, Gehalt(HPLC): 89,3 % Dies entsprach einer Ausbeute von 42,4 % bzgl. eingesetztem Vince-Lactam.

## Patentansprüche

1. Verfahren zur Herstellung von (1 S,4R)-4-(2-Amino-6-chlor-9-H-purin-9-yl)-2-cyclopenten-1-methanol, oder dessen Salzen, der Formel 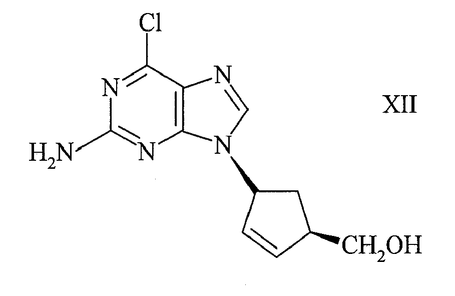 **dadurch gekennzeichnet, dass** man (-)-2-Azabicyclo[2.2.1]hept-5-en-3-on oder (-)-Acyl-2-azabicyclo[2.2.1]hept-5-en-3-on der Formeln 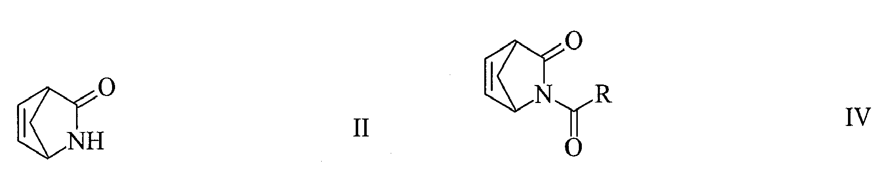 worin R C₁₋₄-Alkyl, C₁₋₄-Alkoxy, Aryl oder Aryloxy bedeutet, mit einem Metallhydrid in einen Aminoalkohol der Formel 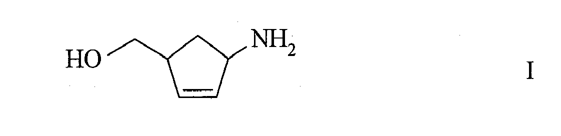 oder in ein Cyclopentenderivat der allgemeinen Formel 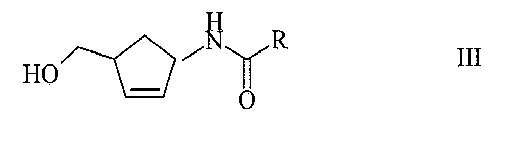 worin R die genannte Bedeutung hat, reduziert, diese dann in die entsprechenden Hydrohalogenidsalze überführt, dann mit N-(2-Amino-4,6-dichlorpyrimidin)-5-yl)formamid der Formel 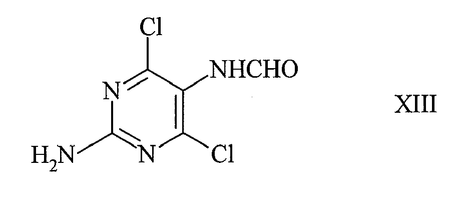 zum (1S,4R)-4-[(2-Amino-6-chlor-5-formamido-4-pyrimidinyl)-amino]-2-cyclopenten-1-methanol der Formel 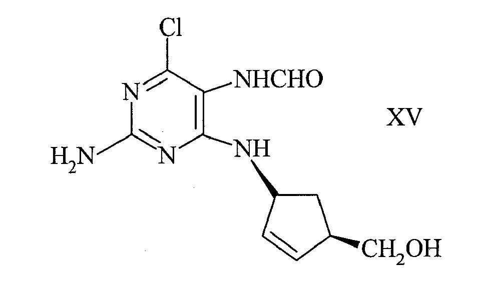 umsetzt und letzteres auf bekannte Weise zu den Verbindungen der Formel XII cyclisiert.
